# FASCICULE DE BREVET EUROPEEN

(11) **EP 4 072 615 B1**
(45) Date de publication et mention de la délivrance du brevet: **15.10.2025**
(21) Numéro de dépôt: 20819786.3
(22) Date de dépôt: 09.12.2020
(51) Int. Cl.: A61M 1/36, A61M 39/22

(54) **DISPOSITIF D'INTERFACE ENTRE UN EQUIPEMENT EXTERNE ET DES TUBES DESTINES A ETRE RELIES A UN SYSTEME D'UN PATIENT**
SCHNITTSTELLENVORRICHTUNG ZWISCHEN EINEM EXTERNEN GERÄT UND LEITUNGEN, DIE AN EIN PATIENTENSYSTEM ANGESCHLOSSEN WERDEN SOLLEN
INTERFACE DEVICE BETWEEN AN EXTERNAL PIECE OF EQUIPMENT AND LINES INTENDED TO BE CONNECTED TO A PATIENT SYSTEM

(30) Priorité: 13.12.2019 FR 1914441
(43) Date de publication de la demande: 19.10.2022
(73) Titulaire: Saeptum, 14200 Herouville-Saint- Clair (FR)
(72) Inventeur: JEAN, Eric, 14112 BIEVILLE-BEUVILLE (FR); MISSAIRE, Fabrice, 1428 LILLOIS (BE); THUAUDET, Sylvain, 14480 FRESNE-CAMILLY (FR)
(74) Mandataire: Cabinet Boettcher
(86) Numéro de dépôt international: PCT/EP2020/085364
(87) Numéro de publication internationale: WO 2021/122242

(56) Documents cités:
- EP-A1- 3 643 342
- WO-A1-2009/001152
- WO-A2-2005/046439
- US-A- 5 894 011
- US-A1- 2005 145 549
- US-B1- 6 596 234

## Description

L'invention concerne le domaine du dispositif d'interface entre d'une part un équipement externe tel qu'une machine à hémodialyse, une réserve de fluide nutritionnel ou une réserve de fluide médicamenteux et d'autre part au moins un tube destiné à être reliés à un système d'un patient tel qu'un système circulatoire ou un système digestif.

### ARRIERE PLAN DE L'INVENTION

Certaines pathologies nécessitent l'injection de fluide vers un système du patient.

L'implantation du ou des tubes utilisés pour réaliser une injection est traumatisante pour le patient.

Ainsi, lorsqu'une série d'injections doit être réalisée sur une période de plusieurs jours / plusieurs semaines / plusieurs mois, on préfère laisser le / les tubes connectés avec le système du patient pour qu'ils soient réutilisables pour plusieurs injections successives.

La liaison entre l'équipement externe et le patient se fait alors via au moins un tube veineux et via un dispositif d'interface qui sert de connecteur entre l'équipement externe et le tube qui débouche au niveau du système du patient.

Entre deux injections, le / les tube(s) qui restent implantés sur le patient doivent être maintenus dans un environnement limitant le risque de développement d'éléments pathogènes.

Pour cela, après avoir réalisé l'injection, un fluide de verrouillage est injecté dans chaque tube.

Ce fluide de verrouillage permet d'éviter un bouchage du /des tubes qui débouchent dans le système du patient et le besoin de remplacement de tube / tubes / cathéter.

Le cas particulier de l'hémodialyse va maintenant être présenté.

L'hémodialyse consiste en la circulation du sang d'un patient vers un équipement externe de type machine à hémodialyse, puis après avoir traité le sang à l'aide de la machine à hémodialyse, le sang est restitué vers le système circulatoire du patient.

Pour mettre en œuvre la liaison entre la machine à dialyse et le système circulatoire du patient, le personnel médical relie un premier conduit à la machine à hémodialyse et fait circuler un liquide dans ce premier conduit pour en chasser les gaz.

Une fois le premier conduit purgé de ses gaz, il est alors relié mécaniquement, via un dispositif d'interface audit au moins un tube / cathéter préalablement implanté sur le patient (chaque au moins un tube / cathéter débouche dans le système circulatoire du patient).

L'aspiration de sang vers la machine à hémodialyse peut alors commencer.

En fin de séance d'hémodialyse, le cathéter relié au patient est débranché de la machine et du sérum physiologique y est injecté pour restituer le sang se trouvant dans ce cathéter vers le système circulatoire du patient et rincer le tube.

Enfin, chaque au moins un tube est relié à un moyen d'injection de fluide de verrouillage et du fluide de verrouillage est injecté dans chaque tube / cathéter.

Entre deux séances d'hémodialyse, le fluide de verrouillage présent dans chaque tube / cathéter permet de limiter le risque de développement de pathogènes / infection via le tube.

Ce fluide de verrouillage sera retiré lorsque l'on voudra réaliser une nouvelle hémodialyse.

Toutes ces opérations manuportées sont reproduites à chaque fois que l'on veut relier le patient à la machine à hémodialyse via un cathéter. Ainsi, dans les cas où l'on implante un cathéter veineux pour admettre du sang vers le patient et un cathéter artériel pour prélever du sang du patient, on doit multiplier les opérations manuportées. Le risque de contamination du système circulatoire du patient s'en trouve augmenté. Des dispositifs d'interface pour réaliser une hémodialyse en utilisant deux cathéters sont présentés dans les documents US5894011, US2005/145549, US6596234, WO2005/046439 and WO2009/001152.

Un dispositif d'interface pour réaliser une hémodialyse est présenté sur le document brevet US5713850A.

Ce dispositif permet d'échanger du fluide avec le patient via un seul tube relié au patient.

Par conséquent, tant pour l'injection de fluide nutritionnel vers un système du patient (selon le cas système digestif ou circulatoire) que pour l'injection de fluide médicamenteux ou l'injection de fluide de dialyse vers le système circulatoire, il serait utile de développer un dispositif d'interface minimisant le risque de contamination du système du patient.

### OBJET DE L'INVENTION

Un objet de la présente invention est de fournir un dispositif d'interface entre un équipement externe et au moins un tube veineux résolvant tout ou partie des inconvénients précités de l'art antérieur.

### RESUME DE L'INVENTION

A cette fin, il est proposé selon l'invention un dispositif d'interface entre un équipement externe et au moins un tube veineux destiné à être relié à un système du patient pour transférer du fluide de l'équipement externe vers le système du patient, ce dispositif d'interface comprenant au moins :
- un premier port adapté à être relié à un port de sortie de l'équipement externe ;
- un port veineux pour injecter du fluide vers le tube veineux ;
le dispositif d'interface étant adapté à sélectivement adopter une première configuration dans laquelle le passage de fluide entre le premier port et le port veineux est interdit et une deuxième configuration dans laquelle le premier port est relié au port veineux pour permettre le passage de fluide du premier port vers le port veineux.

Le dispositif d'interface selon l'invention est essentiellement caractérisé :
- d'une part en ce qu'il comporte un troisième port et en ce qu'il est adapté pour sélectivement adopter une configuration d'accès verrou veineux distincte desdites première et deuxième configurations, dans cette configuration d'accès verrou veineux le troisième port est relié au port veineux alors que le premier port est isolé vis-à-vis du port veineux ; et
- d'autre part en ce que le port veineux est porté par un stator du dispositif d'interface, le premier port et le troisième port étant portés par un ensemble mobile du dispositif d'interface qui est mobile par rapport au stator, le dispositif d'interface étant agencé pour passer d'une de ses configurations vers une autre de ses configurations par déplacement relatif de l'ensemble mobile par rapport au stator.

Le fait d'avoir au moins des premier port et troisième ports portés par un ensemble mobile du dispositif (préférentiellement l'ensemble mobile est un rotor) et au moins un port veineux porté par le stator du dispositif, permet de faciliter l'utilisation du dispositif d'interface puisque :
- d'une part, les ports destinés à être reliés avec l'équipement externe sont portés par un ensemble mobile qui les regroupe alors que ledit au moins un port veineux destiné être relié vers le patient via le tube veineux est porté par le stator ; et puisque
- d'autre part, c'est le déplacement de l'ensemble mobile par rapport au stator qui permet de commander le passage du dispositif d'interface de l'une de ses configurations sélectives vers une autre de ses configurations sélectives.

Grâce au dispositif d'interface selon l'invention, il n'est plus nécessaire de successivement :
- brancher manuellement sur le tube veineux, un port d'aspiration de liquide de verrouillage appartenant à l'équipement externe (pour retirer le fluide de verrouillage contenu dans le tube veineux) ; puis
- débrancher manuellement ce port d'aspiration pour permettre le branchement manuel sur le tube veineux d'un port de sortie de l'équipement externe (pour injecter du fluide de l'équipement externe vers le tube veineux) ; puis
- débrancher manuellement ce port de sortie de l'équipement externe pour permettre le branchement manuel sur le tube veineux d'un port d'injection de liquide de verrouillage vers le tube veineux.

En réduisant le nombre de branchements / débranchements manuels nécessaires de ports vis-à-vis du tube veineux, on réduit grandement le risque d'erreurs de manipulation et le risque associé de contamination du patient.

Le dispositif d'interface selon l'invention peut être utilisé pour :
- former une interface entre un équipement externe de type réserve de fluide nutritionnel et le tube veineux, dans ce cas le tube veineux débouche dans le système circulatoire ou digestif du patient pour y injecter une solution fluide nutritionnel ; ou
   - former une interface entre un équipement externe de type réserve de fluide médicamenteux et le tube veineux, dans ce cas le tube veineux débouche dans le système circulatoire du patient pour y injecter une solution fluide médicamenteuse ; ou
   - former une interface entre un équipement externe comportant une machine à hémodialyse et le tube veineux, dans ce cas le tube veineux débouche dans le système circulatoire du patient pour échanger du sang entre la machine à hémodialyse et le système circulatoire du patient.

Selon un mode de réalisation particulier, le dispositif d'interface selon l'invention comprend aussi un deuxième port préférentiellement adapté à être relié à un port d'entrée de l'équipement externe (en l'occurrence au port d'entrée d'une machine à hémodialyse lorsque cet équipement externe est une machine à hémodialyse), ce deuxième port étant porté par ledit ensemble mobile du dispositif d'interface et dans ladite configuration d'accès verrou veineux le premier port et le deuxième port (Pm2) sont respectivement isolés vis-à-vis du port veineux.

Selon un mode de réalisation particulier du dispositif d'interface selon l'invention, l'ensemble mobile est un rotor monté à rotation relativement audit stator.

En montant l'ensemble mobile à rotation par rapport au stator on peut aisément fixer des jeux fonctionnels entre des surfaces fonctionnelles du rotor et du stator nécessaires à l'étanchéité et la mobilité relative rotor / stator.

L'ensemble mobile peut aussi comporter une face visible depuis l'extérieur du dispositif d'interface et chacun des ports portés par cet ensemble mobile débouche sur cette face.

Comme ces ports de l'ensemble mobile sont regroupés et accessibles depuis une même face cela facilite leurs liaisons avec les ports de l'équipement externe.

Préférentiellement, cette face de l'ensemble mobile et les ports portés par cet ensemble mobile sont intégralement placée à l'intérieur d'un évidement du stator.

Ainsi, cette face et les ports de l'ensemble mobile sont protégés dans l'évidement du stator.

Préférentiellement, les ports portés par l'ensemble mobile sont des ports femelles qui débouchent suivant une direction commune à ces ports femelles.

Ainsi chacune des liaisons avec ces ports femelles se fait simplement par déplacement suivant ladite direction commune Dx.

Selon un mode de réalisation particulier, le dispositif d'interface selon l'invention est également adapté à former une interface entre l'équipement externe, qui comprend une machine à hémodialyse, et un tube artériel destiné à être relié audit système du patient pour transférer du fluide du système du patient vers la machine à hémodialyse, ce dispositif d'interface comprenant également :
- un deuxième port adapté à être relié à un port d'entrée de la machine à hémodialyse, ce deuxième port étant porté par ledit ensemble mobile du dispositif d'interface ;
- un port artériel porté par le stator pour recevoir du fluide du patient (en l'occurrence le fluide est du sang du patient) provenant du tube artériel ;
le dispositif d'interface étant en outre adapté pour interdire le passage de fluide entre le deuxième port et le port artériel lorsque le dispositif se trouve dans sa première configuration et pour autoriser le passage de fluide entre le deuxième port et le port artériel lorsque le dispositif se trouve dans sa deuxième configuration.

Le dispositif d'interface permet de relier des tubes veineux et artériel à une même machine à hémodialyse tout en permettant, à l'aide de ce seul dispositif d'interface, de :
- soit simultanément interdire le passage de fluide entre la machine à hémodialyse et le tube veineux et entre la machine à hémodialyse et le tube artériel en plaçant le dispositif d'interface dans sa première configuration ;
- soit simultanément autoriser le passage de fluide entre la machine à hémodialyse et le tube veineux et entre la machine à hémodialyse et le tube artériel en plaçant le dispositif d'interface dans sa deuxième configuration.

On peut ainsi, avec un même dispositif d'interface, sélectivement autoriser ou interdire une liaison fluidique veineuse et une liaison fluidique artérielle.

Ceci facilite grandement les manipulations pour le personnel médical tout en réduisant le risque d'erreurs de manipulation.

Pour la compréhension de la présente invention, sauf indication contraire tout port du dispositif d'interface qui n'est pas explicitement mentionné comme relié à un autre port du dispositif d'interface doit être considéré comme isolé vis-à-vis de tous les autres ports du dispositif d'interface.

Par ailleurs, lorsque l'on indique que des ports donnés sont reliés entre eux, cela signifie qu'il y a une communication fluidique entre ces ports donnés.

De même, lorsque l'on indique que deux ports donnés sont isolés l'un de l'autre, cela signifie qu'il n'existe pas de communication fluidique entre ces ports donnés.

Le fluide transitant via le dispositif d'interface est un liquide. Par exemple, ce fluide peut être du sang, un dialysat, du sérum physiologique, un fluide médicamenteux (médicament sous forme liquide), un fluide nutritionnel (solution nutritionnelle).

Selon un autre aspect, l'invention concerne un ensemble d'interfaçage comportant un dispositif d'interface selon l'un quelconque des modes de réalisation de l'invention et une connectique d'interface comportant une prise et une pluralité de tubes souples présentant chacun une extrémité reliée à la prise et une extrémité portant au moins un raccord de connexion (il s'agit d'au moins un raccord propre à ce seul tube), chaque raccord de connexion donné étant destiné à connecter fluidiquement le tube souple portant ce raccord de connexion donné à l'un desdits ports de l'équipement externe qui correspond à ce raccord de connexion donné, la prise étant agencée pour être reliée mécaniquement et de manière amovible audit dispositif d'interface de telle manière que lorsque la prise est reliée mécaniquement audit dispositif d'interface, chacun des tubes souples de la pluralité de tube souples est alors fluidiquement relié à un seul des ports portés par ledit ensemble mobile qui lui correspond.

Cet aspect de l'invention est particulièrement avantageux car il permet de connecter via une unique prise comportant plusieurs ports l'ensemble des ports d'un équipement externe à l'ensemble des ports portés par l'ensemble mobile du dispositif d'interface.

On facilite ainsi la liaison entre l'équipement externe et le dispositif d'interface puisque l'opérateur n'a pas à connecter chaque port de l'équipement externe directement sur le port correspondant du dispositif d'interface, la connectique d'interface dotée de tubes souples forme une sorte de pieuvre dotée de plusieurs raccords indépendant les uns des autres et donc plus faciles à manipuler.

Une fois que les ports de l'équipement externe sont respectivement connectés aux raccords de la connectique d'interface, l'opérateur n'a plus qu'à brancher la prise présentant de multiples ports sur le dispositif d'interface qui est côté patient.

En facilitant ces opérations, on réduit le risque d'erreurs de branchement et on permet un gain de temps pour l'opérateur.

Préférentiellement la connectique d'interface comporte un bouchon agencé pour être monté de manière amovible sur la prise de la connectique d'interface, ce bouchon définissant un volume interne étanche entre la prise et le bouchon pour que chacun des tubes souples reliés à la prise puisse être mis en communication fluidique avec les autres tubes souples via ce volume interne au bouchon.

Ainsi, avant de relier la prise de la connectique d'interface audit dispositif d'interface, on peut facilement évacuer les gaz contenus dans les tubes souples en circulant du liquide dans l'ensemble de ces tubes qui sont reliés entre eux grâce au volume interne du bouchon.

Ceci contribue à réduire le temps nécessaire à l'établissement d'une liaison fluidique fiable entre l'équipement externe et le système du patient.

Selon un autre aspect, l'invention concerne un système d'hémodialyse comprenant un ensemble d'interface selon l'un quelconque des modes de réalisation de l'invention et un équipement externe qui comprend une machine à hémodialyse.

Dans ce système d'hémodialyse, le premier port du dispositif d'interface est relié de manière amovible au port de sortie de la machine à hémodialyse via la connectique d'interface et le deuxième port du dispositif d'interface est relié de manière amovible au port d'entrée de la machine à hémodialyse via la connectique d'interface.

La machine à hémodialyse comporte une pompe agencée pour faire circuler des fluides de son port d'entrée vers son port de sortie et le système d'hémodialyse comprend également un tube veineux et un tube artériel.

Le tube veineux est relié au port veineux du dispositif d'interface. Ce tube veineux est ici destiné à être relié au système circulatoire d'un patient pour transférer, via le dispositif d'interface et via la connectique d'interface, du sang de la machine à hémodialyse vers le système circulatoire.

Le tube artériel est relié au port artériel du dispositif d'interface. Ce tube artériel est ici destiné à être relié audit système circulatoire du patient pour transférer, via le dispositif d'interface et via la connectique d'interface, du sang du système circulatoire vers la machine à hémodialyse.

Le système d'hémodialyse selon l'invention est avantageux au moins pour les raisons énoncées ci-avant en référence au dispositif d'interface selon l'invention. Enfin, suivant un autre aspect, l'invention concerne une connectique d'interface pour relier un équipement externe à un dispositif d'interface pour injecter un fluide vers un patient, caractérisée en ce que cette connectique comportant une prise et une pluralité de tubes souples présentant chacun une extrémité reliée à la prise et une extrémité portant au moins un raccord de connexion, chaque raccord de connexion donné étant destiné à connecter fluidiquement le tube souple portant ce raccord de connexion donné à un port de l'équipement externe qui correspond à ce raccord de connexion donné, la prise présentant une pluralité de ports mâles débouchant chacun vers une face de la prise et chacun desdits tubes souples étant relié fluidiquement à un seul desdits ports mâles qui lui correspond et inversement, chacun desdits ports mâles étant relié à un seul desdits tubes souples qui lui correspond.

Cette connectique permet de regrouper plusieurs ports mâles sur une même face de la prise pour permettre un branchement fluidique simultané de ces ports mâles vers des ports correspondants appartenant à un dispositif d'interface.

### BREVE DESCRIPTION DES DESSINS

D'autres caractéristiques et avantages de l'invention ressortiront clairement de la description qui en est faite ci-après, à titre indicatif et nullement limitatif, en référence aux dessins annexés, dans lesquels:

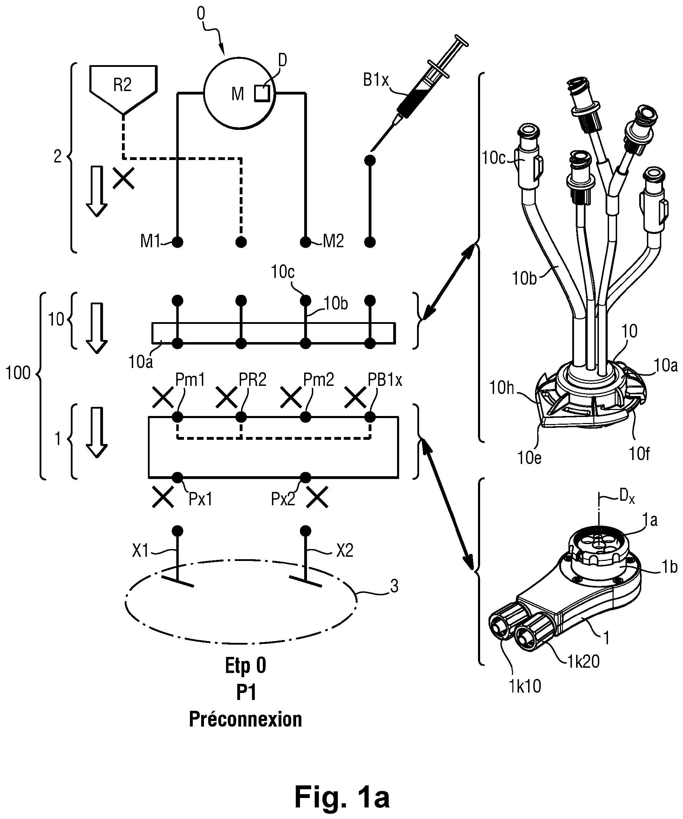

[Fig. 1a] la figure 1a présente un système d'hémodialyse 0 selon l'invention comprenant une machine à hémodialyse 2, un dispositif d'interface 1 selon l'invention, une connectique d'interface 10 selon l'invention (ce dispositif d'interface 1 et la connectique d'interface 10 formant un ensemble d'interfaçage 100 selon l'invention) et des tubes veineux et artériel X1, X2, ce dispositif d'interface 1, les tubes veineux et artériel X1, X2, les ports d'entrée et de sortie M1, M2 de la machine et la connectique d'interface sont ici représentés avant d'être assemblés entre eux pour transférer du fluide entre l'équipement externe 2 et la système 3 du patient ;

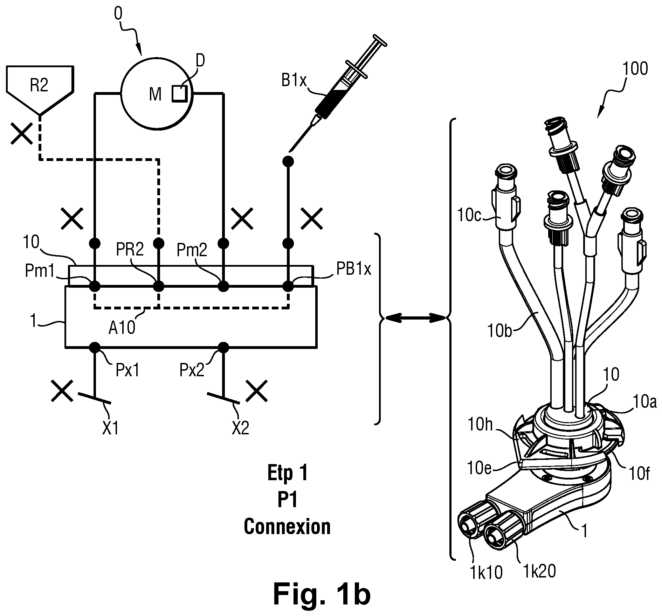

[Fig. 1b] la figure 1b illustre l'ensemble des éléments de la figure 1a mais ils sont ici reliés les uns aux autres pour transférer du fluide entre l'équipement externe 2 et la système 3 du patient, le dispositif d'interface est ici dans sa première configuration P1, Etp1 où les ports veineux Px1 et artériel Px2 sont obturés (ce dispositif d'interface 1 est ici équipé d'une coque externe car il est destiné à être implanté en extracorporel et non à être fixé sur un os du patient) ;

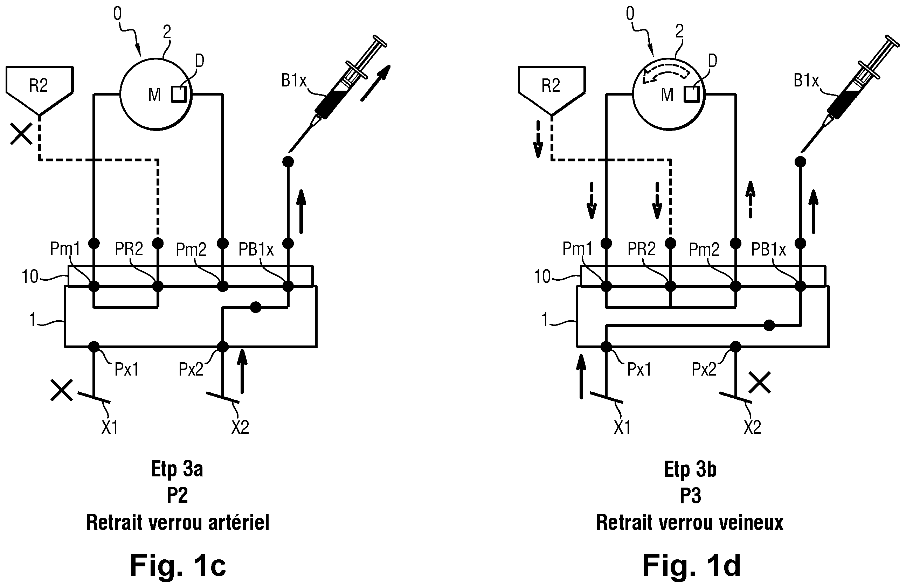

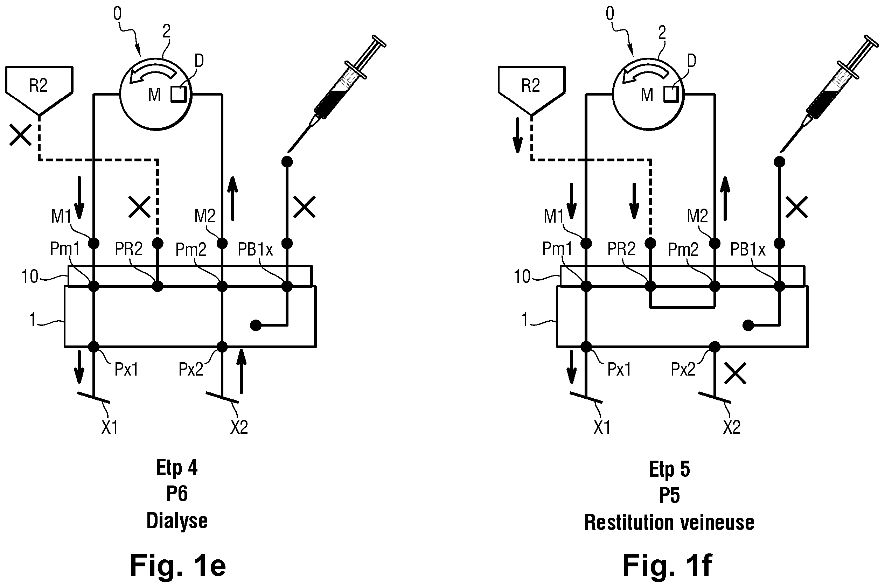

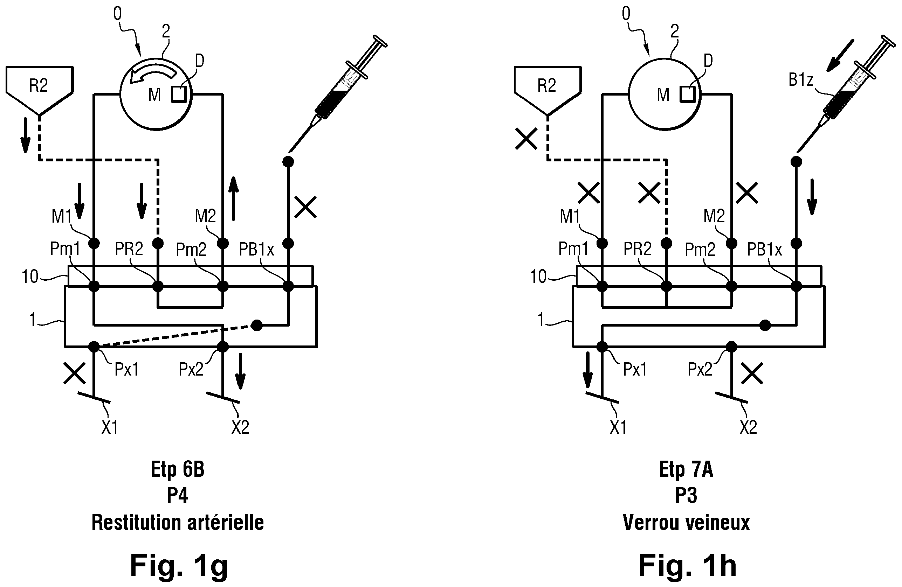

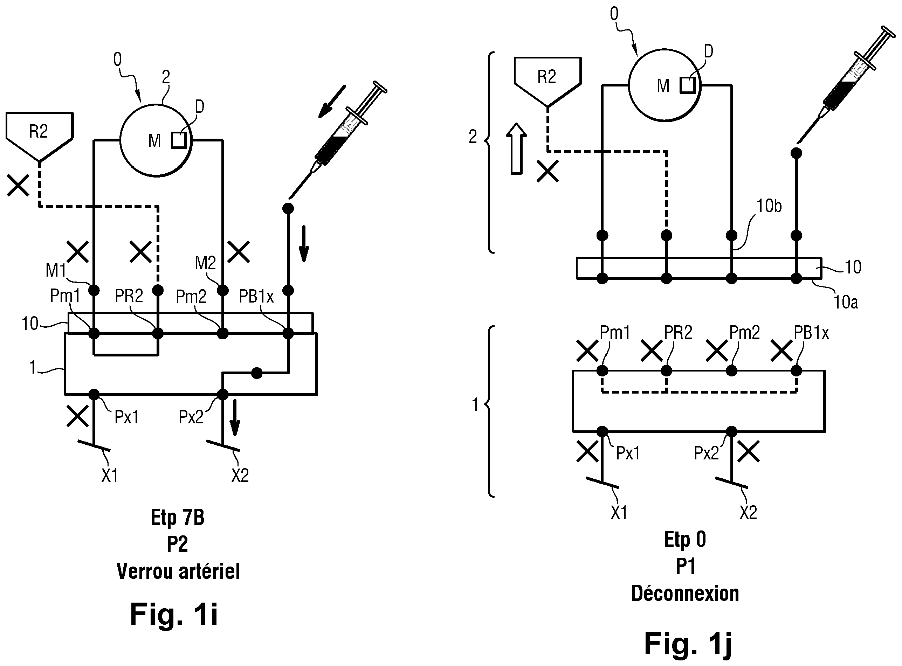

[Fig. 1c] ; [Fig. 1d] ; [Fig. 1d] ; [Fig. 1e] ; [Fig. 1f] ; [Fig. 1g] ; [Fig. 1h] ; [Fig. 1i] ; [Fig. 1j] les figures 1b, 1c, 1d, 1e, 1f, 1g, 1h, 1i, 1j illustrent respectivement les différentes étapes de fonctionnement successives Etp1, Etp3a, Etp3b, Etp4, Etp5, Etp6B, Etp7A, Etp7B, Etp0 d'un dispositif d'interface 1 selon l'invention adapté à l'hémodialyse ;

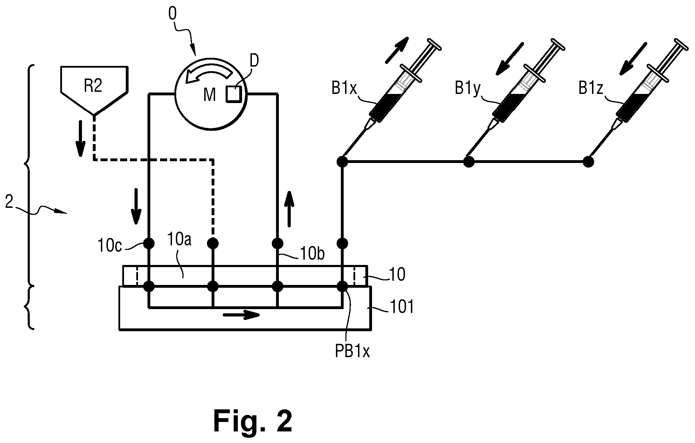

[Fig. 2] la figure 2 illustre la connectique d'interface 10 selon l'invention alors qu'elle est reliée à l'équipement externe 2 et qu'un bouchon 101 est fixé sur la prise afin de mettre en relation les tubes souples 10b de la connectique les uns avec les autres via un ce volume interne au bouchon (on peut ainsi remplir les tubes souples 10b de fluide liquide et en évacuer des gaz) ;

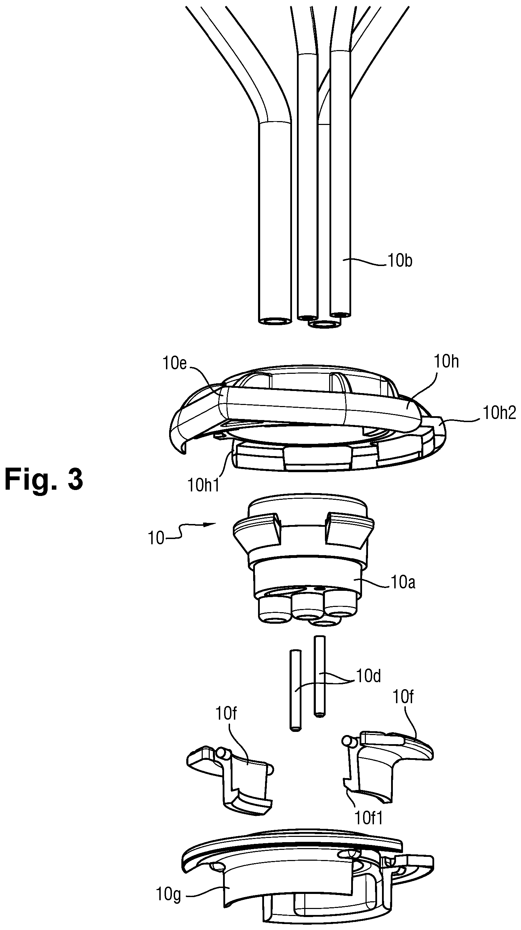

[Fig. 3] la figure 3 illustre une vue éclatée de la connectique d'interface 10 selon l'invention ;

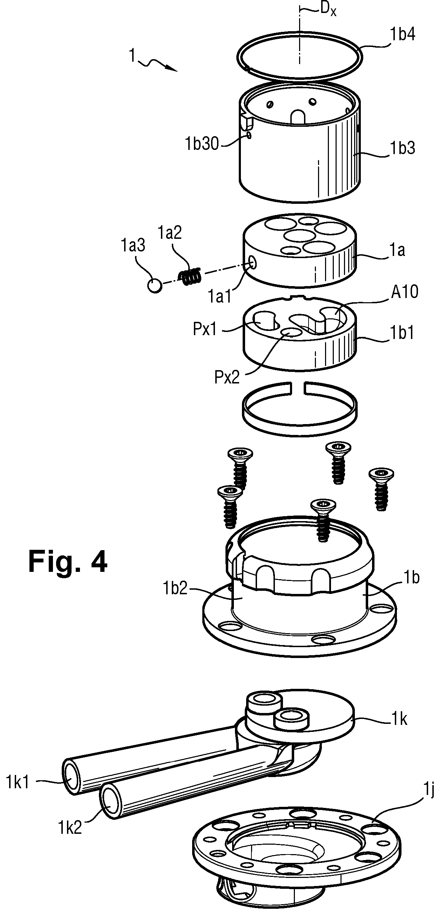

[Fig. 4] la figure 4 illustre une vue éclatée du dispositif d'interface 1 selon un mode particulier de l'invention (ici, ce dispositif possède une base agencée pour être fixée sur un os du patient ;

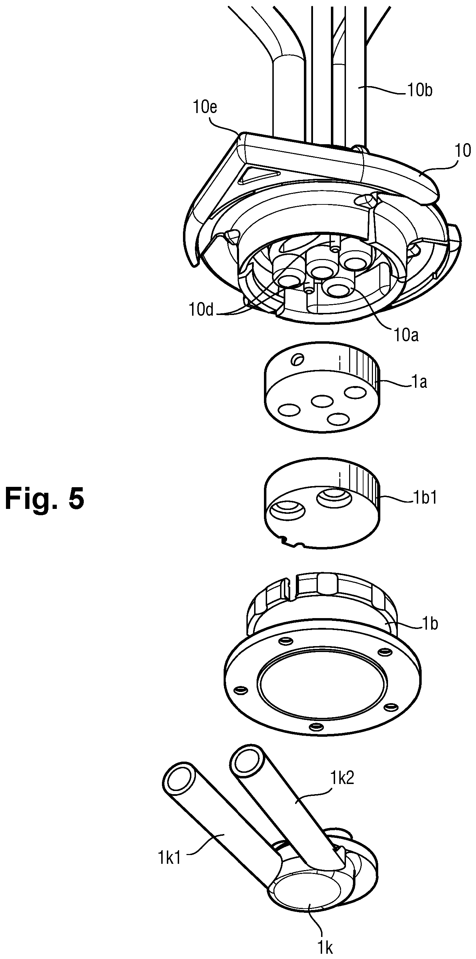

[Fig. 5] la figure 5 illustre une première vue éclatée d'un ensemble d'interface 100 selon l'invention comprenant le dispositif d'interface 1 et la connectique d'interface 10 ;

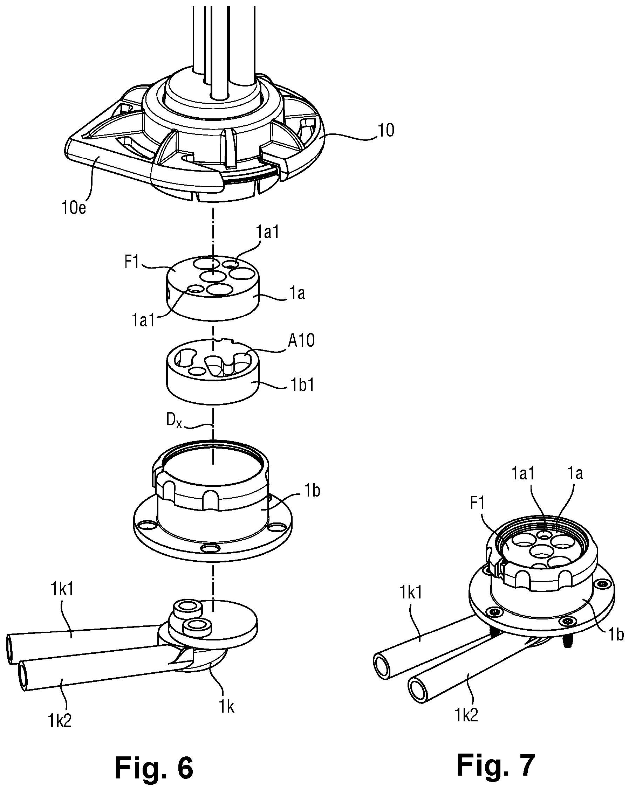

[Fig. 6] la figure 6 illustre une seconde vue éclatée de l'ensemble d'interface 100 de la figure 5 ;
[Fig. 7] la figure 7 illustre un dispositif d'interface selon l'invention adapté pour être fixé sur un os du patient ;

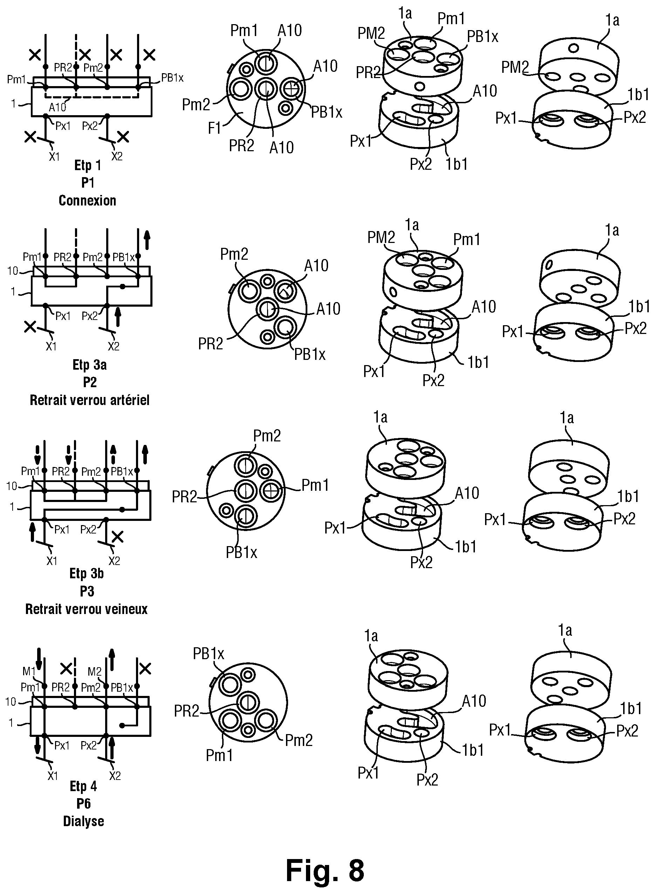

[Fig. 8] la figure 8 illustre un dispositif d'interface 1 selon l'invention dans plusieurs configurations successives qui sont ici la première configuration P1 (adoptée à l'étape Etp1), la configuration d'accès verrou artériel P2 (adoptée à l'étape Etp3a pour retirer le verrou artériel contenu dans le tube artériel X2), la configuration d'accès verrou veineux P3 (adoptée à l'étape Etp3b pour retirer le verrou veineux contenu dans le tube veineux X1), la deuxième configuration (P6 adoptée à l'étape Etp4 pour réaliser la dialyse) ;

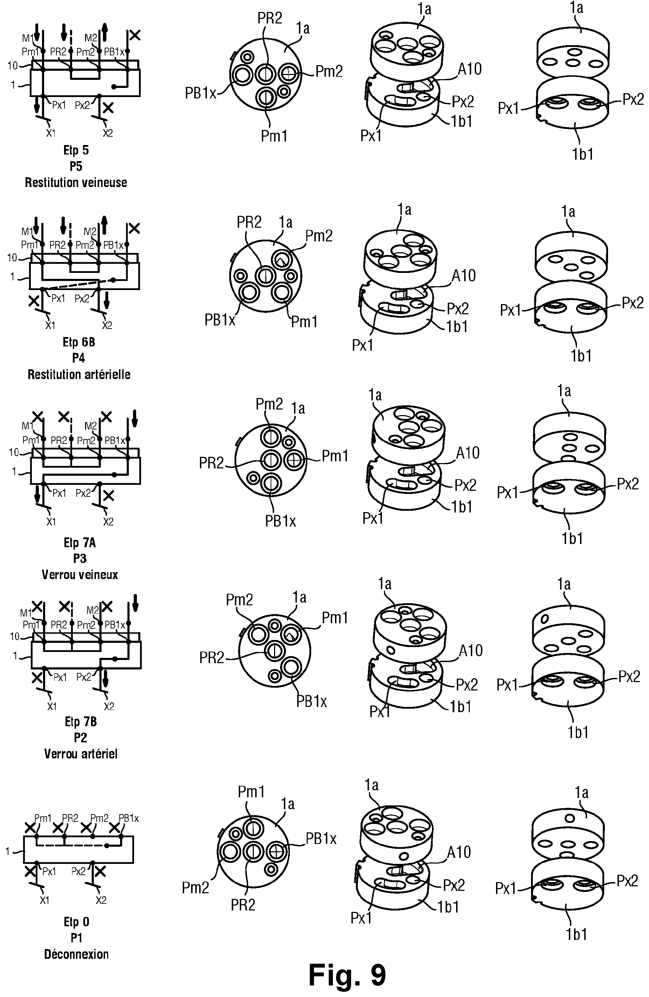

[Fig. 9] la figure 9 illustre un dispositif d'interface 1 selon l'invention dans plusieurs configurations successives qui sont ici la configuration de restitution veineuse P5 (adoptée à l'étape Etp5, mise en œuvre à la fin de la dialyse pour restituer du sang et/ou du dialysat vers le patient via le tube veineux X1 où un verrou veineux va pouvoir être placé, cette configuration P5 est adoptée après que le dispositif 1 soit passé dans sa seconde configuration P6 pour réaliser la dialyse), la configuration de restitution artérielle P4 (adoptée à l'étape Etp6B mise en œuvre pour restituer du sang et/ou du dialysat vers le patient via le tube artériel X2 où un verrou artériel va pouvoir être placé, cette configuration P4 est adoptée après que le dispositif 1 soit passé dans sa seconde configuration P6 pour réaliser la dialyse et préférentiellement après qu'il ait adopté la configuration P5 et réalisé la restitution veineuse), puis on passe à l'étape Etp7A de mise en place du verrou veineux qui est réalisée avec le dispositif 1 placé en configuration d'accès verrou veineux P3, puis on passe à l'étape Etp7B de mise en place du verrou artériel qui est réalisée avec le dispositif 1 placé en configuration d'accès verrou artériel P2, puis on passe à l'étape Etp0 pour déconnecter la connectique 10 et l'équipement externe 2 vis-à-vis du dispositif d'interface 1 qui peut rester à demeure sur le patient (soit en extracorporel soit sous forme d'implant transcutané) en restant relié aux tubes veineux X1 et artériel X2.

### DESCRIPTION DETAILLEE DE L'INVENTION

L'invention porte principalement sur un système d'hémodialyse 0 illustré au travers des figures 1a à 1j.

Ce système 0 comprend un équipement externe 2 qui comprend une machine à hémodialyse 2, un ensemble d'interface 100 selon l'invention et des tubes veineux et artériel X1, X2.

L'ensemble d'interface 100 comprend un dispositif d'interface 1 selon l'invention et une connectique d'interface 10 selon l'invention.

Le dispositif d'interface selon l'invention est prévu pour d'un côté permettre la fixation et la liaison fluidique avec les tubes X1, X2 et pour d'un autre côté permettre le branchement de la connectique 10 directement sur le dispositif d'interface pour pouvoir établir des liaisons fluidiques entre l'équipement externe 2 et le système circulatoire 3 du patient via la connectique 10 et le dispositif d'interface 10 qui sont ainsi en série entre le patient et l'équipement externe.

Ces tubes X1, X2 sont chacun agencés pour être mis en communication fluidique avec le système circulatoire 3 d'un patient. Ces tubes veineux X1 et artériel X2 appartiennent préférentiellement à un même cathéter.

Chacun des tubes X1, X2 est préférentiellement formé dans un conduit souple, par exemple thermoplastique compatible avec un usage médical (polyuréthane, PEEK, silicone ou autre), pour faciliter sa manipulation et son cheminement entre le système circulatoire 3 et le dispositif d'interface 1.

Le tube veineux X1 est essentiellement destiné à l'injection de fluide de la machine 2 vers le patient, et le tube artériel X2 est essentiellement destiné au prélèvement (aspiration) de fluide du patient vers la machine à hémodialyse 2.

La mise en relation des tubes avec le système circulatoire 3 se fait soit via une fistule artérioveineuse, via un cathéter central tunnélisé dans le patient (un cathéter central tunnelisé est un cathéter restant en place dans le corps du patient entre deux hémodialyses) ou via une implantation d'un cathéter d'hémodialyse non tunnélisé (ces tubes pouvant appartenir à un ou plusieurs cathéters).

Plus précisément, le dispositif d'interface 1 selon l'invention forme :
- une interface entre un équipement externe, ici la machine à hémodialyse 2, et un tube artériel X2 destiné à être relié audit système circulatoire 3 du patient pour transférer du fluide / sang du système 3 vers la machine à hémodialyse 2 ; et
- une interface entre la machine à hémodialyse 2 et au moins un tube veineux X1 pour transférer du fluide / sang de la machine à hémodialyse vers le système circulatoire 3.

Ce dispositif d'interface 1 comprend :
- un premier port Pm1 adapté à être relié, préférentiellement de manière amovible via un premier raccord, à un port de sortie M1 de la machine à hémodialyse 2 ; et
- un deuxième port Pm2 adapté à être relié, préférentiellement de manière amovible via un second raccord éventuellement solidarisé audit premier raccord, à un port d'entrée M2 de la machine à hémodialyse ;
- un port veineux Px1 pour injecter du sang vers le tube veineux X1, ce port veineux Px1 étant préférentiellement attaché de manière amovible, via un raccord, au tube veineux X1 ; et
- un port artériel Px2 pour recevoir (faire circuler, prélever par exemple par aspiration) du sang du patient provenant du tube artériel X2, ce port artériel Px2 est préférentiellement attaché de manière amovible, via un raccord, au tube artériel X2.

Le deuxième port Pm2 du dispositif d'interface 1 est adapté à transférer du sang du dispositif d'interface 1 vers un port d'entrée M2 de la machine à hémodialyse 2.

La machine à hémodialyse comporte une pompe M agencée pour pomper / faire circuler des fluides de son port d'entrée M2 vers son port de sortie M1.

Le premier port Pm1 du dispositif d'interface 1 est adapté à recevoir du sang provenant du port de sortie M1 pour le transférer vers le tube veineux X1.

La machine à hémodialyse 2 est aussi adaptée à réaliser des échanges entre le fluide qu'elle transfert, en l'occurrence du sang du patient, et un dialysat liquide pour permettre une épuration du fluide (sang). Pour cela la machine à hémodialyse comporte un circuit interne relié d'un côté au port d'entrée M2 et d'un autre côté au port de sortie M1.

La pompe M de la machine à hémodialyse est préférentiellement une pompe péristaltique, pour transférer du fluide du port d'entrée M2 vers le port de sortie M1 avec un débit précisément contrôlé.

Ce circuit interne de la machine à hémodialyse comporte préférentiellement au moins une membrane semi-perméable permettant de réaliser des échanges entre le fluide / le sang et le dialysat formulé chimiquement et/ou des filtres pour épurer le fluide / sang.

Préférentiellement, cette membrane semi-perméable permet des échanges entre un circuit où passe le sang du patient et un circuit de dialysat non représenté. Le circuit de dialysat s'étend depuis une réserve de dialysat préalablement formulé vers une réserve de dialysat usagé en passant par une zone de contact contre la membrane semi-perméable pour réaliser les échanges avec le sang du patient.

La machine à hémodialyse 2 comporte aussi un débulleur D destiné à retirer des bulles de gaz contenues dans le fluide transféré via la machine à hémodialyse 2. Ici, le débulleur D est relié en série entre les ports d'entrée M2 et de sortie M1 et il se trouve préférentiellement entre le port M2 et la membrane. Si besoin, la machine peut comporter d'autres débulleurs pour s'assurer de ne pas diffuser de liquide contenant des bulles vers le patient.

La machine à hémodialyse peut aussi comporter un dispositif de détection pour détecter des bulles et/ou des impuretés et/ou un débit de fluide entre ses ports d'entrée M2 et de sortie M1 et/ou une pression du fluide transitant via la machine 2 et/ou un niveau de dialysat dans une réserve de dialysat reliée à la machine 2 pour amener ce dialysat au contact de la membrane semi-perméable.

Ce dispositif de détection est relié à une unité électronique de commande (non représentée) de la machine à hémodialyse 2 pour commander le fonctionnement de la pompe M en fonction de mesures réalisées par ce dispositif de détection.

Cette unité électronique peut aussi être reliée à certains au moins desdits capteurs du circuit de dialysat et à des actionneurs du circuit de dialysat pour par exemple de commander des débits de dialysat dans le circuit de dialysat, des dosages de dialysat avec d'autres constituants.

Cette unité électronique peut aussi être reliée à un ou plusieurs capteurs d'état de la membrane semi-perméable pour commander les paramètres de fonctionnement des différents actionneurs dont la pompe M en fonction de mesures réalisées à l'aide de ce/ces capteur(s) d'état.

La machine à hémodialyse peut aussi comporter une interface de communication (non représentée) adaptée à détecter une configuration courante du dispositif d'interface 1 selon l'invention de manière à ajuster le fonctionnement de la machine à hémodialyse en fonction de la configuration courante ainsi détectée. Cette interface de communication peut comprendre une connectique électronique reliant, de manière détachable, la machine à hémodialyse 2 au dispositif d'interface 1.

Cette interface de communication peut être adaptée pour transmettre :
- du dispositif d'interface 1 vers la machine à hémodialyse 2 un signal de configuration courante du dispositif d'interface 1 représentatif d'une configuration courante adoptée par ce dispositif d'interface 1 ; et/ou
- de la machine à hémodialyse 2 vers le dispositif d'interface 1, un signal de changement de configuration, le dispositif d'interface 1 comportant un actionneur, par exemple un moteur, commandant le changement de configuration du dispositif d'interface 1 en fonction du signal de changement de configuration reçu par le dispositif d'interface 1 de manière à faire passer le dispositif d'interface d'une configuration courante vers une autre configuration choisie dans une succession de configurations prédéfinies. Les différentes configurations sélectivement adoptées par le dispositif d'interface seront présentées ci-après.

Les figures 1a à 1j, 8 et 9 illustrent une succession de configurations que peut adopter sélectivement le dispositif d'interface (par sélectivement, on entend que le dispositif d'interface ne peut adopter à un instant donné qu'une seule configuration parmi les configurations listées).

Le dispositif d'interface 1 comporte un troisième port PBlx et est adapté pour sélectivement adopter une configuration d'accès verrou veineux P3 ; Etp3b distincte desdites première et deuxième configurations P1, P6 respectivement adoptées aux étapes Etp1 ; Etp4.

Dans cette configuration d'accès verrou veineux P3 (étape Etp3b) le troisième port Pblx est relié au port veineux Px1 alors que le premier port Pm1 et le deuxième port Pm2 sont respectivement isolés vis-à-vis du port veineux Px1.

Comme on le comprend des figures 4 à 8, le port veineux Px1 est porté par un stator 1b du dispositif d'interface 1 et le premier port Pm1 et le troisième port PBlx sont portés par un ensemble mobile 1a qui est ici un rotor 1a monté à rotation relativement au stator 1b.

Cet ensemble mobile 1a est mobile par rapport au stator 1b sur une plage éventuellement limitée par des butées.

Le dispositif d'interface 1 est agencé pour passer d'une de ses configurations vers une autre de ses configurations par déplacement relatif de l'ensemble mobile 1a par rapport au stator 1b.

Plus particulièrement, l'ensemble mobile 1a comporte une face F1 visible depuis l'extérieur du dispositif d'interface 1 et chacun des ports portés par cet ensemble mobile 1a débouche sur cette face.

La liaison fluidique entre un port porté par le rotor 1a et un port porté par le stator 1b présente l'intérêt de n'avoir qu'une seule zone de frottement entre le stator et l'ensemble mobile sur le chemin de cette liaison fluidique.

Ceci est avantageux puisque cela permet de simplifier la conception du dispositif en réduisant au minimum, le nombre de points d'étanchéité dynamique à développer sur le chemin fluidique entre un port porté par l'ensemble mobile 1a et un port porté par le stator. On minimise ainsi le risque d'avoir des zones de rétention de matière sur le chemin entre les ports de l'ensemble mobile et les ports du stator 1b.

Cette conception permet aussi de compacter le dispositif puisque plusieurs ports peuvent être portés sur une seule et même pièce mobile.

Préférentiellement, la face (F1) de l'ensemble mobile (1a) et les ports portés par cet ensemble mobile sont intégralement placée à l'intérieur d'un évidement du stator.

Les ports portés par l'ensemble mobile 1a sont préférentiellement des ports femelles qui débouchent suivant une direction Dx commune à ces ports femelles.

Cela permet d'une part de compacter la pièce mobile 1a tout en permettant un accès vers ces ports femelles par une translation unique suivant la direction Dx.

Chaque configuration donnée du dispositif, est ainsi définie par une position donnée de la pièce mobile 1a vis-à-vis du stator 1b.

Comme on le comprend des figures 1a, 1e et 8, le dispositif d'interface 1 est adapté à sélectivement adopter :
- une première configuration P1 adoptée aux étapes de pré-connexion Etp0, de connexion Etp1 et de déconnexion ; et
- une deuxième configuration P6 adoptée lors de l'étape de dialyse Etp4.

Dans sa première configuration P1 :
le passage de sang entre le premier port Pm1 et le port veineux Px1 est interdit ; et
le passage de sang entre le deuxième port Pm2 et le port artériel Px2 est interdit.

Cette première configuration est utile au moins pour isoler la machine 2 vis-à-vis du système circulatoire du patient. Dans sa deuxième configuration P6 (étape de dialyse Etp4 illustrée à la figure 1e), le premier port Pm1 est relié au port veineux Px1 pour permettre le passage de sang du premier port Pm1 vers le port veineux Px1, le deuxième port Pm2 et le port artériel Px2 sont aussi reliés entre eux pour permettre le passage de sang du port artériel Px2 vers le deuxième port Pm2. Dans cette deuxième configuration P6, seuls les ports Pm1, Px1, Pm2 Px2 sont ouverts et les autres ports du dispositif sont fermés.

Cette deuxième configuration P6 est utile pour faire circuler le sang en boucle en passant successivement par le système circulatoire 3, le tube artériel X2, le port artériel Px2, le deuxième port Pm2, le port d'entrée M2, la machine à hémodialyse 2 et son circuit interne, le port de sortie M1, le deuxième port Pm1, le port veineux Px1, le tube veineux X1 et finalement le système circulatoire 3.

Un des avantages du dispositif d'interface 1 selon l'invention est qu'il permet de passer d'une configuration à un autre sans avoir à débrancher manuellement un port de la machine et par simple déplacement du rotor 1a.

On peut ainsi autoriser ou interrompre le passage de fluide entre les tubes X1, X2 et la machine à hémodialyse tout en limitant les risques de contamination du patient.

Dans un mode de réalisation préférentiel, le dispositif d'interface peut être agencé pour que lorsqu'il se trouve dans sa première configuration P1, le premier port Pm1 et l'un au moins des autres ports PR2, PBlx portés par l'ensemble mobile 1a soient alors mis en communication, via un circuit interne A10 au dispositif d'interface 1.

Dans ce même mode, lorsque le dispositif d'interface 1 se trouve dans sa deuxième configuration P6, la communication entre le premier port Pm1 et les autres ports (PR2, PB1x, Pm2) portés par l'ensemble mobile (1a) via ce circuit interne A10 est alors interdite.

Comme on le voit sur les figures 8 et 9, ce circuit interne A10 est formé dans le stator et débouche uniquement en face du rotor 1a.

Ainsi, dans la première configuration, on peut mettre en communication les premier port Pm1 avec un ou plusieurs autres ports PR2, PBlx portés par l'ensemble mobile pour permettre un éventuel dégazage de certains au moins des ports de l'ensemble mobile et d'au moins des portions de la connectique 10 tout en isolant les tubes artériel et veineux X2, X1.

Le dispositif d'interface 2 est en outre adapté à sélectivement adopter une configuration de d'accès verrou artériel P2 distincte desdites première et deuxième configurations P1, P6 et de la configuration d'accès verrou veineux P3.

Dans cette configuration d'accès verrou artériel P2 (adoptée à l'étape Etp3a visible aux figures 1c, 1i, le troisième port Pblx est relié au port artériel Px2 alors que le premier port Pm1 et le deuxième port Pm2 sont respectivement isolés vis-à-vis du port artériel Px2, les ports veineux et artériel Px1, Px2 étant alors également isolés l'un de l'autre.

Par ports isolés les uns des autres, isolés l'un de l'autre, on entend que ces ports ne communiquent pas fluidiquement entre eux.

Cette configuration d'accès verrou artériel P2 permet, via le troisième port Pblx, l'aspiration ou l'injection d'un fluide de verrouillage depuis ou vers tube artériel X2 pour selon le cas, soit libérer ce tube artériel et y autoriser le passage de sang / la circulation de fluide liquide, soit le verrouiller pour s'opposer au passage de sang vers ce tube artériel X2.

Un fluide de verrouillage est une substance ayant une fonction anticoagulante et une fonction de tampon pour éviter le passage de sang dans le tube qui le contient. Eventuellement, le fluide de verrouillage peut avoir une fonction antiseptique.

Typiquement, après avoir réalisé une hémodialyse, du fluide de verrouillage est injecté dans chaque tube devant rester dans le patient jusqu'à l'hémodialyse suivante. Ce fluide de verrouillage permet d'éviter un bouchage du tube et le besoin de le remplacer.

Grâce au dispositif d'interface 1, on peut injecter ou aspirer du fluide de verrouillage vers le tube veineux X1 et/ou le tube artériel X2 tout en laissant ce(s) tube(s) connecté(s) au dispositif d'interface 1.

Encore une fois, on réduit ainsi le besoin de manipulation des raccords et ports et les risques associés pour le patient.

Afin d'aspirer du fluide de verrouillage provenant du tube veineux X1 ou du tube artériel X2, le système selon l'invention peut aussi comporter au moins une seringue d'aspiration B1x de fluide de verrouillage reliée au troisième port Pblx via la connectique d'interface (10).

Il est à noter que le port de la connectique d'interface 10 destiné à être relié au troisième port Pblx présente un tube souple pouvant être relié à deux raccords placés en parallèle comme sur la figure 1a ou à trois raccords placés en parallèles comme sur la figure 2, chacun de ces raccords pouvant recevoir une seringue comme la seringue B1x pour aspirer du fluide de verrouillage ou une seringue B1z destinée à injecter du fluide de verrouillage pour verrouiller les tubes X1, X2 ou une seringue d'injection de sérum physiologique B1y pour rincer les tubes X1, X2.

Dans le mode de réalisation présenté aux figures 1a à 1j, on constate que le troisième port PBlx est prévu pour être connecté soit au port veineux Px1, soit au port artériel Px2 soit éventuellement à un port de restitution Pr2 destiné à injecter un fluide à restituer au patient (par exemple un sérum physiologique ou médicament liquide). Ainsi, ce même troisième port Px1 est utilisable avec chaque port veineux Px1 et artériel Px2 pour réaliser l'insertion d'un verrou ou son aspiration. Pour cela, ce troisième port PBlx peut être prévu pour être relié à tour de rôle avec une seringue d'aspiration B1x ou avec une seringue d'injection B1z de fluide de verrouillage. Le couplage de chaque seringue peut être effectué manuellement par le praticien d'hémodialyse.

Cette solution est aussi avantageuse puisqu'elle permet de réaliser des opérations de verrouillage des ports veineux ou artériel à l'aide de ce seul troisième port Pblx.

Pour réaliser un verrouillage veineux ou artériel, il suffit de disposer le dispositif d'interface en configuration d'accès verrou veineux ou artériel et d'injecter un verrou artériel ou veineux via celui des ports Px1 ou Px2 relié au troisième port Pblx.

Le dispositif d'interface selon l'invention permet ainsi de réaliser les opérations de retrait ou de mise en place de fluide de verrouillage dans le tube veineux X1 et le tube artériel X2 tout en laissant la machine à hémodialyse 2 et les tubes veineux et artériel X1, X2 connectés / reliés aux ports concernés du dispositif d'interface. Encore une fois, on limite le risque d'avoir une contamination du système circulatoire par des branchements ou débranchements de ports.

A cette fin, le système selon l'invention peut comporter une seringue d'injection artérielle Blzde fluide de verrouillage reliée au troisième port Pblx pour pouvoir injecter du fluide de verrouillage vers le tube artériel X2.

Selon un mode de réalisation préférentiel, le dispositif d'interface comporte également un port de restitution Pr2 porté par ledit ensemble mobile 1a et le dispositif d'interface 1 est en outre adapté pour sélectivement adopter une configuration de restitution veineuse P5 (adoptée à l'étape Etp5) distincte autres configurations pouvant être adoptées par le dispositif P1, P6, P3.

Dans cette configuration de restitution veineuse P5, le port de restitution Pr2 est relié audit deuxième port Pm2 (en l'occurrence, cette liaison se fait via ledit circuit interne A10 formé dans le stator visible aux figures 4, 6, 8, 9) pour pouvoir injecter un fluide de restitution vers la machine à hémodialyse 2, le premier port Pm1 étant alors relié audit port veineux Px1 et isolé de tous les autres ports du dispositif d'interface, et le port artériel Px2 étant au moins isolé vis-à-vis du port veineux Px1, du premier port Pm1 et du deuxième port Pm2.

Dans cette configuration de restitution veineuse P5 (adoptée à l'étape Etp5 illustrée aux figures 1f, 9), le dispositif 1 permet de faire passer un fluide à restituer (fluide de restitution) vers le seul deuxième port Pm2 de la machine à hémodialyse tout en permettant à cette machine à hémodialyse d'injecter du fluide vers le tube veineux X1 via le port de sortie M1 mis en communication avec le premier port Pm1 et le port veineux Px1.

A cette fin, le système selon l'invention peut comporter un appareil R2 pour alimenter en fluide à restituer (par exemple une réserve de sérum physiologique, un dialysat, un médicament sous forme liquide) reliée au port de restitution Pr2 via la connectique 10 pour pouvoir injecter du fluide à restituer vers le dispositif d'interface 1 lorsqu'il se trouve dans sa configuration de restitution veineuse P5, Etp5.

Comme la machine à hémodialyse 2 forme un circuit s'étendant entre son port d'entrée M2 et son port de sortie M1, la machine à hémodialyse pompe le fluide de restitution de son port d'entrée M2 vers son port de sortie M1 pour ainsi pousser le sang contenu dans la machine et le restituer vers le tube veineux X1 puis vers le système circulatoire 3 du patient.

Ainsi, le volume de sang contenu dans la machine à hémodialyse 2, dans le tube veineux X1 et dans les conduites reliant la machine à hémodialyse 2 au dispositif d'interface 1 peut être restitué au patient. Ceci est particulièrement important pour limiter la quantité de sang perdue par le patient durant l'hémodialyse.

Selon un mode de réalisation préférentiel, le dispositif 1 est en outre adapté pour sélectivement adopter une configuration de restitution artérielle P4 à l'étape Etp6B distincte des autres configurations que peut adopter le dispositif d'interface P1, P6, P2, P3, P5.

Dans cette configuration de restitution artérielle P4 (voir étape ETp6B aux figures 1g et 9), le port de restitution Pr2 est relié audit deuxième port Pm2 (en l'occurrence, cette liaison se fait via ledit circuit interne A10 formé dans le stator) pour pouvoir injecter un fluide de restitution vers l'équipement externe 2, le premier port Pm1 étant alors relié audit port artériel Px2 et isolé de tous les autres ports du dispositif d'interface, et le port veineux Px1 étant au moins isolé vis-à-vis du port artériel Px2, du premier port Pm1 et du deuxième port Pm2.

Dans cette configuration de restitution artérielle P4 à l'étape Etp6B, le fluide de restitution transite du port de restitution Pr2 vers le deuxième port Pm2, puis ce fluide de restitution transite via la machine M et son débulleur D pour en ressortir et transiter du premier port Pm1 vers le port artériel Px2 (le dispositif d'interface 1 en configuration de restitution artérielle relient ses port Pm1 et Px2 entre eux en les isolant des autres ports du dispositif d'interface).

Dans un mode de réalisation non illustré, il est aussi possible qu'en configuration de restitution les deux ports veineux et artériel soient reliés au port de restitution Pr2 ou éventuellement au premier port Pm1 pour réaliser simultanément la restitution. Cette solution permettrait un gain de temps lors de la restitution mais elle présente l'inconvénient de ne pas contrôler le volume de fluide restitué à chaque tube X1, X2.

Comme indiqué précédemment, ce fluide de restitution peut être du sérum physiologique.

Dans cette configuration de restitution artérielle P4, le tube artériel X2 est rempli de fluide à restituer et le sang présent dans ce tube X2 est poussé vers le système circulatoire 3.

Cette restitution de sang limite la perte de sang lors de l'hémodialyse et évite le risque de colmatage du tube artériel X2.

Dans un mode de réalisation particulier, le dispositif d'interface peut être adapté pour que lorsqu'il se trouve dans sa première configuration P1 (à l'étape Etp0 et/ou à l'étape Etp1), son port de restitution Pr2 soit alors relié à l'un au moins desdits du premier port Pm1 et du deuxième port Pm2.

Ainsi, les premier et deuxième ports Pm1, Pm2 peuvent être dégazés en injectant un fluide (liquide) via le port de restitution Pr2.

Dans un mode particulier, le dispositif d'interface 1 peut comporter un mécanisme de commande motorisé (non représenté) agencé pour déplacer l'ensemble mobile 1a par rapport au stator 1b et ainsi commander le passage du dispositif 1 de l'une de ses configurations vers une autre de ses configurations selon une succession de configurations prédéfinie.

Par exemples le mécanisme de commande peut agir soit directement sur l'ensemble mobile soit indirectement via un déplacement de la prise 10.

En d'autres termes, le mécanisme de commande permet de commander le passage d'une configuration courante dans laquelle se trouve le dispositif vers une autre configuration du dispositif sélectionnée, cette autre configuration étant sélectionnée parmi les différentes configurations que peut sélectivement adopter le dispositif.

L'enchainement des différentes configurations nécessaires à la réalisation d'une hémodialyse complète sera détaillé plus loin.

Comme indiqué précédemment, la liaison entre la machine 2 et le dispositif d'interface se fait via une connectique d'interface 10 comportant une prise 10a et une pluralité de tubes souples 10b présentant chacun une extrémité reliée à la prise 10a et une autre extrémité portant au moins un raccord de connexion 10c (chaque raccord de connexion est porté par un seul des tubes souples de la connectique auquel il correspond, un tel raccord de connexion peut être mâle ou femelle, par exemple de type standard LUER).

Chaque raccord de connexion 10c donné est destiné à connecter fluidiquement le tube souple portant ce raccord de connexion donné 10c à l'un desdits ports de l'équipement externe 2 qui correspond à ce raccord de connexion donné 10c.

La prise 10a étant agencée pour être reliée mécaniquement et de manière amovible audit dispositif d'interface 1 de telle manière que lorsque la prise 10a est reliée mécaniquement audit dispositif d'interface 1, chacun des tubes souples 10b de la pluralité de tube souples est alors fluidiquement relié à un seul des ports portés par ledit ensemble mobile 1a qui lui correspond.

Les raccords de connexion 10c peuvent être déplacés les uns par rapport aux autres dans la limite de la liberté autorisée par les tubes souples 10b.

La prise 10a de la connectique 10 et l'ensemble mobile 1a du dispositif d'interface 1 sont conformées pour que lorsque la prise 10a est reliée mécaniquement audit dispositif d'interface 1, cette prise (10a) est alors assujettie en déplacement avec le rotor / ensemble mobile 1a lors du déplacement de ce rotor 1a par rapport au stator 1b.

Ainsi, lorsque la prise 10a est reliée mécaniquement audit dispositif d'interface 1, l'utilisateur peut déplacer la prise 10a par rapport au stator 1b et ainsi entraîner le déplacement de l'ensemble mobile 1a par rapport à ce stator 1b pour commander le passage du dispositif d'interface 1 de l'une de ses configurations vers une autre de ses configurations.

Pour permettre cet assujettissement en déplacement de la prise 10a par rapport à l'ensemble mobile 1a, deux broches 10d sont portées par la prise 10a et deux évidements 1a1 complémentaires de ces broches 10d sont réalisés dans ledit ensemble mobile 1a.

Ces broches 10d et les évidements 1a1 sont prévus pour que la prise 10a ne puisse s'assembler sur l'ensemble mobile 1a que dans une seule position d'orientation de la prise 10a par rapport à l'ensemble mobile 1a. Ainsi, les broches 10d et les évidements 1a1 forment un détrompeur.

Préférentiellement, ces broches 10d ont des longueurs suffisantes pour que chacune de ces broches 10d puisse pénétrer dans l'un de ces évidements 1a1 qui lui correspond alors que le reste de la prise 10a est maintenu à distance du dispositif d'interface 1.

Ainsi, ces broches 10d facilitent le pré positionnement et le guidage de la prise 10a par rapport à l'ensemble mobile pendant que la prise est approchée du dispositif d'interface 1 pour relier mécaniquement la prise 10a au dispositif d'interface 1.

La connectique d'interface 10 comporte aussi un indicateur 10e de position de la prise 10a, visible depuis l'extérieur de la connectique d'interface 10, pour informer un opérateur d'une position courante de la prise par rapport au stator 1b lorsque la prise 10a est reliée mécaniquement audit dispositif d'interface 1.

En l'occurrence, comme illustré sur la figure 1a, 1b, 3, 5, 6, cet indicateur 10e est formé par un index s'étendant radialement par rapport à un axe longitudinal de la prise.

Cet index est accessible depuis l'extérieur de l'ensemble d'interfaçage 100 pour permettre à l'opérateur de manipuler l'index et ainsi déplacer la prise 10a par rapport ou stator lorsque la prise est reliée mécaniquement audit dispositif d'interface 1.

Préférentiellement, la connectique d'interface 10 comporte au moins un verrou mécanique 10f agencé :
- pour interdire l'écartement relatif de la prise 10a vis-à-vis du dispositif d'interface 1 lorsque la prise 10a est reliée mécaniquement audit dispositif d'interface 1 et que cette prise 10a est éloignée d'une position relative prédéterminée de la prise 10a par rapport au stator 1b ; et
- pour autoriser l'écartement relatif de la prise 10a vis-à-vis du dispositif d'interface 1 lorsque la prise 10a est reliée mécaniquement audit dispositif d'interface 1 et que cette prise 10a se trouve dans ladite position relative prédéterminée de la prise 10a par rapport au stator 1b.

Ainsi, cet au moins un verrou 10f permet d'autoriser l'accouplement et le désaccouplement mécanique de la prise 10a vis-à-vis du dispositif d'interface 1, uniquement lorsque cette prise 10a est dans une position prédéterminée par rapport au stator 1b.

Comme la position de la prise 10a est indexée vis-à-vis de l'ensemble mobile 1a (par exemple via les broches 10d) et que la position de l'ensemble mobile vis-à-vis du stator 1b détermine la configuration courante adoptée par le dispositif d'interface 1, le fait d'autoriser l'accouplement ou désaccouplement de la prise 10a sur le dispositif d'interface 1 uniquement lorsque cette prise est dans une position prédéterminée vis-à-vis du stator permet de garantir que cet accouplement ou désaccouplement se fasse toujours lorsque le dispositif d'interface 1 est dans une configuration donnée prédéfinie.

En l'occurrence, ledit au moins un verrou mécanique 10f est agencé pour autoriser l'écartement relatif de la prise 10a vis-à-vis du dispositif d'interface 1 uniquement lorsque le dispositif d'interface 1 se trouve dans sa première configuration P1 ; Etp1, le passage de fluide entre les ports Pm1, PR2, Pm2, PBlx et chacun des ports veineux Px1 et artériel Px2 étant alors interdit.

Ainsi, la connexion ou la déconnexion de la prise 10a ne peut intervenir que lorsque ces ports veineux et artériels Px1, Px2 sont sécurisés pour interdire toute circulation de fluide via les tubes veineux et artériel X1, X2.

Comme on le comprend de la figure 3, qui représente la connectique d'interface 10, cette connectique d'interface comporte un disque de connexion 10g entourant la prise 10a.

Ce disque de connexion 10g est conformé pour :
- d'une part être indexé à rotation vis-à-vis du stator 1b lorsque la prise 10a est reliée mécaniquement audit dispositif d'interface 1 ; et pour
- d'autre part autoriser le pivotement de ce disque de connexion 10g par rapport à la prise 10a qui s'étend à l'intérieur du disque de connexion 10g.

Le verrou comporte deux bras 10f disposés de part et d'autre du disque de connexion 10g et montés pivotants par rapport à ce disque de connexion 10g qui entoure la prise 10a.

Chacun de ces bras présente un ergot 10f1 à l'une de ses extrémités, chaque ergot étant disposé pour pénétrer dans une gorge annulaire 1b2 formée sur le stator 1b lorsque la prise 10a est reliée mécaniquement audit dispositif d'interface 1.

Chacun de ces ergots 10f1 positionné à l'intérieur de la gorge annulaire 1b2 du stator s'oppose à l'écartement de la prise 10a vis-à-vis du dispositif d'interface 1.

Ainsi, pour autoriser l'écartement de la prise 10a vis-à-vis du dispositif d'interface 1, il faut préalablement sortir les ergots 10f1 de la gorge annulaire 1b2 et pour cela, il faut pivoter les bras 10f par rapport au disque de connexion 10g.

Comme on le comprend des figures 1a, 1b et 3, la connectique d'interface 10 comporte un anneau indicateur 10h portant ledit index 10e.

Cet anneau indicateur 10h est assujetti mécaniquement sur la prise 10a pour tourner avec cette prise 10a lors du déplacement de la prise 10a vis-à-vis du stator 1b.

Cet anneau indicateur 10h comporte deux découpes 10h1, 10h2 qui permettent respectivement un accès vers chacun des bras 10f lorsque la prise 10a est dans une première position angulaire prédéterminée vis-à-vis du disque de connexion 10g qui porte ces bras 10f.

Cet anneau indicateur 10h est agencé pour interdire le pivotement des bras 10f par rapport au disque de connexion 10g lorsque cet anneau indicateur 10h se trouve dans une autre position angulaire (vis-à-vis du disque de connexion 10g) que ladite première position angulaire prédéterminée vis-à-vis du disque de connexion 10g.

Ainsi, l'accouplement de la prise vis-à-vis du dispositif d'interface 1 n'est possible que si :
- si d'une part la prise 10a est dans une position angulaire prédéterminée vis-à-vis de l'ensemble mobile 1a ; et
- si d'autre part les bras 10f sont libres de pivoter vis-à-vis du disque de connexion 10g, c'est à sire si la prise 10a et l'anneau indicateur 10h sont dans ladite première position angulaire prédéterminée vis-à-vis du disque de connexion 10g.

Préférentiellement, la prise 10a présente une pluralité de ports mâles chacun orienté pour pénétrer dans un des ports Pm1, PR2, Pm2, PBlx qui lui correspond et qui est porté par l'ensemble mobile 1a, réalisant ainsi une pluralité de connexions fluidiques entre les ports mâles portés par la prise 10a et les ports correspondants portés par l'ensemble mobile 1a.

Préférentiellement ladite prise 10a et lesdits tubes souples sont en matériaux polymères alors que l'un au moins parmi l'ensemble mobile 1a et le stator 1b est au moins partiellement constitué en un matériau métallique.

L'usage de matériaux polymères permet d'obtenir une connectique d'interface 10 amovible de faible coût par rapport au coût du dispositif d'interface 1 qui présente au moins une pièce métallique plus résistante et par conséquent plus couteuse.

Le matériau métallique peut être choisi parmi un alliage inox, un alliage de titane ou autre alliage métallique biocompatible.

Ainsi, la connectique d'interface 10 peut être prévue à usage unique (la connectique 10 est un consommable) alors que le dispositif d'interface 1 est prévu pour un usage multiple.

Il est à noter que le stator du dispositif d'interface 1 peut présenter une embase métallique 1j prévue pour être implantée sur un os du patient et y rester à demeure.

Une telle embase métallique 1j peut, comme sur les figures 4, 5, 6 et 7 présenter des passages pour des vis pour fixer l'embase 1j par vissage dans un os du patient.

Comme on le comprend de ces mêmes figures 4 à 7, le stator du dispositif d'interface 1 peut aussi présenter un insert 1k prévu pour définir des premières et seconde voies de passage de fluide 1k1, 1k2 qui sont coudées.

La première de ces voies 1k1 présente une première extrémité débouchant en vis-à-vis d'un premier passage réalisé au travers d'un disque 1b1 de stator 1b se trouvant dans une enceinte interne 1b3 du stator 1b pour ainsi former ledit port veineux Px1 du dispositif d'interface 1.

La seconde de ces voies 1k2 présente une première extrémité débouchant en vis-à-vis d'un second passage réalisé au travers du disque 1b1 de stator 1b pour ainsi former ledit port artériel Px2 du dispositif d'interface 1.

La première de ces voies 1k1 présente une seconde extrémité qui est prévue pour être reliée fluidiquement en série avec le tube veineux X1, cette liaison pouvant éventuellement être réalisée par un premier raccord spécifique 1k10 pour être réversible.

De même, la seconde de ces voies 1k2 présente une seconde extrémité qui est prévue pour être reliée fluidiquement en série avec le tube artériel X2, cette liaison pouvant éventuellement être réalisée par un second raccord spécifique 1k20 pour être réversible.

Les voies 1k1 et 1k2 de l'insert 1k sont coudées pour permettre aux tubes veineux et artériel X1, X2 de s'étendre longitudinalement dans un plan perpendiculaire à une direction Dx suivant laquelle s'étendent les ports portés par l'ensemble mobile 1a. on obtient un dispositif plus compacte dont le stator peut être placé contre le patient avec les tube veineux et artériel X1, X2 cheminant le long de l'enveloppe externe du patient alors que les ports portés par l'ensemble mobile 1a s'étendent perpendiculairement à cette enveloppe externe.

Il est ainsi plus aisé de relier la connectique d'interface 10 sur le dispositif d'interface 1 restant à demeure sur le patient. Un capuchon (non représenté) est mis en place sur le dispositif d'interface 1 afin de le protéger entre deux séances (les ports destinés à être reliés vers l'équipement externe 2 sont masqués de manière étanche par ce capuchon).

Comme illustré sur les figures 4 à 7, l'ensemble mobile 1a est préférentiellement un rotor 1a en forme de disque monté rotatif à l'intérieur de ladite enceinte interne 1b3 du stator 1b qui présente un alésage intérieur cylindrique.

Un mécanisme d'indexation à rotation du rotor par rapport à l'enceinte interne 1b3 est proposé sous la forme d'un ressort placé dans un alésage borgne radial 1a1 du rotor, d'un ressort 1a2 placé dans cet alésage 1a1 et d'une bille 1a3 poussée par ce ressort contre une face annulaire interne cylindrique de l'enceinte interne 1b3.

Cette enceinte interne 1b3 est pourvue d'une pluralité de perforation radiales 1b30 de diamètres inférieurs au diamètre de la bille et qui se trouvent sur le chemin parcouru par la bille 1a3 lors de la rotation du rotor.

Le rotor 1a est ainsi indexé à rotation par rapport au stator 1b à chaque fois que la bille pénètre dans une de ces perforations 1b30. Chaque indexation définit une position du rotor qui correspond à une seule desdites configurations pouvant être adoptées par le dispositif d'interface 1.

Le stator 1b comporte un disque 1b1 placé à l'intérieur de ladite enceinte interne 1b3 du stator 1b. Le disque du rotor 1a comporte une surface destinée à venir en compression contre une surface complémentaire appartenant au disque du stator 1b1 de telle manière que l'on ait une étanchéité entre ces surfaces s'opposant à toute fuite de fluide le long de ces surfaces des disques.

Pour permettre cette mise en compression du disque 1b1 de stator 1b contre le disque de rotor 1a, ladite enceinte interne 1b3 du stator 1b porte une butée axiale 1b4, ici formée par un anneau élastique 1b4 contre lequel vient s'appuyer le disque de rotor 1a.

Le disque de stator 1b1 est d'un côté en appui contre le disque de rotor 1a et d'un autre côté contre l'insert 1k qui définit les première et seconde voies de passage de fluide 1k1, 1k2.

La mise en compression de ces disques de rotor et d stator l'un contre l'autre est ici réalisée par l'insert 1k qui est dans un matériau élastiquement compressible tel que du silicone ou du latex.

Ainsi, les disques de stator et de rotor forment un empilement de disque comprimé entre l'anneau élastique 1b4 et l'insert 1K.

Un tel assemblage est intéressant car en retirant l'anneau élastique 1b4 de l'enceinte intérieure 1b3, on peut dépiler le disque de stator 1b1 et le disque de rotor 1a pour réaliser la maintenance du dispositif d'interface 1 et l'éventuel remplacement de ces disques.

Le fonctionnement de l'ensemble 100 selon l'invention va maintenant être présenté.

La figure 2, montre une étape préparatoire dans laquelle la connectique d'interface 10 est équipée d'un bouchon 101 assemblé sur la prise 10a pour définir un espace fermé vers lequel débouchent chacun des tubes souples 10b de la connectique. Cet espace permet de mettre en communication ces tubes souples 10b les uns avec les autres.

Les raccords 10c de cette connectique sont fluidiquement reliés aux ports de l'équipement externe 2, c'est à dire la machine 2, l'appareil R2 (qui permet d'alimenter en liquide comme du sérum physiologique) et la ou les seringues B1x, B1y, B1z.

La pompe de la machine 2 est mise en fonctionnement ce qui permet de faire circuler du liquide en boucle entre la machine 2 et la connectique d'interface 10 en passant par le débulleur D qui permet d'évacuer les bulles du liquide.

La machine 2 réalise cette circulation jusqu'à ce qu'elle détecte, ou qu'un opérateur détecte, que le circuit de la machine et le dispositif d'interface ne contient que du liquide.

Ce liquide est par exemple du sérum physiologique.

Après cette étape préparatoire de la figure 2, on interrompt le fonctionnement de la pompe de la machine, éventuellement on pince des tubes souples, on retire le bouchon 101 et on met en œuvre une première étape (Etp1 illustrée à la figure 1b) consistant à relier mécaniquement les ports de la prise 10a aux ports du dispositif d'interface 1 qui est dans sa première configuration.

Les tubes veineux et artériel X1 et X2 sont ici déjà reliés aux ports correspondants Px1, Px2 de dispositif d'interface 1.

Ainsi, les ports de l'ensemble externe 2 sont reliés au dispositif d'interface via la connectique d'interface 10.

L'appareil R2 qui est relié au port PR2 peut être un port spécifique de la machine à hémodialyse 2 utilisé uniquement lors de la restitution de sang en fin d'hémodialyse ou éventuellement une poche de fluide ou de médicament devant être injectée en fin d'hémodialyse.

On commande alors le passage du dispositif d'interface 1 en configuration d'accès au verrou artériel P2 (étape Etp3a illustrée à la figure 1c).

La seringues d'aspiration B1x est alors actionnée. Le fluide de verrouillage contenu dans le tube artériel X2 est alors aspiré.

L'actionnement de la ou des seringues peut être motorisé et commandé par l'unité de commande de l'équipement externe.

Après aspiration du verrou artériel, la pompe de l'équipement externe 2 est mise en route et le peu de gaz contenu dans le dispositif d'interface est alors évacué vers le débulleur. L'ensemble de la connectique 10 est du dispositif d'interface 1 est maintenant empli de liquide.

On commande alors le passage du dispositif d'interface 1 en configuration d'accès au verrou veineux P3 (étape Etp3b illustrée à la figure 1d).

La seringues d'aspiration B1x ou une autre seringue est alors actionnée. Le fluide de verrouillage contenu dans le tube veineux X1 est alors aspiré.

Une fois les tubes X1, X2 libérés du fluide de verrouillage, on commande le passage du dispositif d'interface dans sa deuxième configuration P6 (étape Etp4). Dans cette deuxième configuration P6, le tube artériel X2 est mis en communication avec le port d'entrée M2 via le port Pm2 et le tube veineux X1 est mis en communication avec le port de sortie M1. Le sang du patient peut alors circuler en boucle via la machine à hémodialyse 2 pour y être dialysé. Le peu de gaz contenu dans le dispositif d'interface est aspiré et évacué par le débulleur D.

Une fois l'hémodialyse effectuée, de manière optionnelle, on peut vouloir restituer un liquide particulier au patient. Dans ce cas, ce liquide peut être amené via un port de restitution PR2 qui appartient au dispositif d'interface. Ce port Pr2 peut être relié à une sortie de sang R2 spécifique de la machine à hémodialyse ou à une poche R2 de sang ou de médicament.

A cette fin, le mécanisme de commande est actionné pour que le dispositif d'interface passe en configuration de restitution veineuse P5 (étape Etp5 illustrée à la figure 1f). Dans cette configuration P5, le port PR2 est mis en communication avec le port Pm2 et le tube veineux X1 est mis en communication avec le port de sortie M1. Le liquide à restituer circule alors de l'appareil R2 jusqu'au tube veineux X1 en passant successivement par le un premier des tubes de la connectique 10, par le port Pr2, par le port Pm2, par un deuxième des tubes de la connectique 10, par le port d'entrée M2, par la machine à hémodialyse 2, par le port de sortie M1, par un troisième des tubes de la connectique 10, par le port Pm1, par le port Px1 et enfin il arrive au tube veineux X1.

Une fois cette étape Etp5 terminée, on commande alors le passage du dispositif d'interface 1 en configuration de restitution artérielle P4 (étape 6B illustrée à la figure 1g) qui autorise :
- la fermeture du port artériel Px2 / du tube artériel X2 (avec une possible liaison au port PB1x) ; et
- la mise en relation de l'appareil périphérique R2 avec le port d'entrée machine M2 via le port de restitution PR2 et via le deuxième port Pm2.

Un dispositif de détection peut détecter l'arrivée du sérum physiologique et ainsi détecter la fin de l'opération de restitution artérielle.

L'opération de restitution est terminée lorsqu'un volume de liquide prédéterminé a été restitué vers le tube artériel X2.

Une fois cette étape 6B terminée, on commande le passage du dispositif d'interface 1 en configuration d'accès verrou veineux P3 (étape Etp7A illustrée à la figure 1h) qui d'une part autorise la mise en communication de la seringue B1z avec le tube veineux X1 via le port PBlx et le port Px1.

Dans cette configuration de verrouillage le port Px2 est fermé.

Le fluide de verrouillage part de la seringue d'injection B1z jusqu'au tube veineux X1.

Une fois cette étape 7A terminée, on commande le passage du dispositif d'interface 1 en configuration d'accès verrou artériel P2 (étape Etp7B illustrée à la figure 1i) qui d'une part autorise la mise en communication de la seringue B1z avec le tube artériel X2 via le port PBlx et le port Px2.

Dans cette configuration d'accès verrou artériel P2, le port Px2 est fermé.

Le fluide de verrouillage part de la seringue d'injection B1z jusqu'au tube artériel X2.

Après avoir réalisé ces injections des verrous, on peut débrancher la connectique 10 vis-à-vis le dispositif d'interface 1, le dispositif d'interface étant alors dans sa première configuration P1 illustrée à l'étape Etp0 de la figure 1j.

Le dispositif d'interface 1 selon l'invention est particulièrement aisé à utiliser puisque le passage de l'une quelconque de ses configurations vers une autre quelconque de ses configurations se fait par simple déplacement relatif de l'ensemble mobile 1a par rapport au stator 1b

## Revendications

1. Dispositif d'interface (1) entre un équipement externe (2) et au moins un tube veineux (X1) destiné à être relié à un système (3) d'un patient pour transférer du fluide de l'équipement vers le système du patient, ce dispositif d'interface (1) comprenant au moins :
- un premier port (Pm1) adapté à être relié à un port de sortie (M1) de l'équipement externe (2);
- un port veineux (Px1) pour injecter du fluide vers le tube veineux (X1) ; le dispositif d'interface étant adapté à sélectivement adopter une première configuration (P1 ; Etp1) dans laquelle le passage de fluide entre le premier port (Pm1) et le port veineux (Px1) est interdit et une deuxième configuration (P6 ; Etp4) dans laquelle le premier port (Pm1) est relié au port veineux (Px1) pour permettre le passage de fluide du premier port (Pm1) vers le port veineux (Px1), **caractérisé en ce que** :
- le dispositif d'interface (1) comporte un troisième port (PB1x) et est adapté pour sélectivement adopter une configuration d'accès verrou veineux (P3 ; Etp3b) distincte desdites première et deuxième configurations (P1, P6 ; Etp1 ; Etp4), dans cette configuration d'accès verrou veineux (P3 ; Etp3b) le troisième port (Pblx) est relié au port veineux (Px1) alors que le premier port (Pm1) est isolé vis-à-vis du port veineux (Px1) ; et **en ce que**
- le port veineux (Px1) est porté par un stator du dispositif d'interface, le premier port (Pm1) et le troisième port (PB1x) étant portés par un ensemble mobile (1a) du dispositif d'interface (1) qui est mobile par rapport au stator (1b), le dispositif d'interface étant agencé pour passer d'une de ses configurations vers une autre de ses configurations par déplacement relatif de l'ensemble mobile (1a) par rapport au stator.

2. Dispositif d'interface (1) selon la revendication 1, dans lequel l'ensemble mobile est un rotor monté à rotation relativement audit stator.

3. Dispositif d'interface (1) selon l'une quelconque des revendications 1 ou 2, dans lequel l'ensemble mobile (1a) comporte une face (F1) visible depuis l'extérieur du dispositif d'interface (1) et chacun des ports portés par cet ensemble mobile (1a) débouche sur cette face.

4. Dispositif d'interface (1) selon la revendication 3, dans lequel ladite face (F1) de l'ensemble mobile (1a) et les ports portés par cet ensemble mobile sont intégralement placée à l'intérieur d'un évidement du stator.

5. Dispositif d'interface selon l'une quelconque des revendications 1 à 4, dans lequel les ports portés par l'ensemble mobile sont des ports femelles qui débouchent suivant une direction (Dx) commune à ces ports femelles.

6. Dispositif d'interface (1) selon l'une quelconque des revendications 1 à 5, comprenant également un deuxième port (Pm2) porté par ledit ensemble mobile du dispositif d'interface (1), le dispositif d'interface étant en outre adapté pour que dans ladite configuration d'accès verrou veineux le premier port (Pm1) et le deuxième port (Pm2) sont respectivement isolés vis-à-vis du port veineux.

7. Dispositif d'interface (1) selon la revendication 6, adapté à former une interface entre l'équipement externe qui comprend une machine à hémodialyse (2) et un tube artériel (X2) destiné à être relié audit système (3) du patient pour transférer du fluide du système du patient vers la machine à hémodialyse, ledit deuxième port (Pm2) étant adapté à être relié à un port d'entrée (M2) de la machine à hémodialyse, le dispositif d'interface (1) comprenant également un port artériel (Px2) porté par le stator pour recevoir du fluide du patient (en l'occurrence le fluide est du sang du patient) provenant du tube artériel (X2) ; le dispositif d'interface (1) étant en outre adapté pour interdire le passage de fluide entre le deuxième port (Pm2) et le port artériel (Px2) lorsque le dispositif se trouve dans sa première configuration (P1; Etp1) et pour autoriser le passage de fluide entre le deuxième port (Pm2) et le port artériel (Px2) lorsque le dispositif se trouve dans sa deuxième configuration (P6 ; Etp4).

8. Dispositif d'interface (1) selon l'une quelconque des revendications 1 à 7, en outre adapté pour mettre en communication, via un circuit interne (A10) au dispositif d'interface (1), le premier port (Pm1) et au moins un autre des port (PR2, PB1x) portés par l'ensemble mobile(1a) lorsque ce dispositif d'interface se trouve dans sa première configuration (P1) et pour interdire la communication entre le premier port (Pm1) et les autres ports (PR2, PB1x, Pm2) portés par l'ensemble mobile (1a) via ce circuit interne (A10) lorsque ce dispositif d'interface se trouve dans sa deuxième configuration (P6 ; Etp4).

9. Dispositif d'interface selon l'une quelconque des revendications 1 à 8 combinée à la revendication 6, adapté pour sélectivement adopter une configuration d'accès verrou artériel (P2 ; Etp3a) distincte desdites première et deuxième configurations (P1, P6 ; Etp1 ; Etp4) et de la configuration d'accès verrou veineux (P3 ; Etp3b), dans cette configuration d'accès verrou artériel (P2 ; Etp3a) le troisième port (Pblx) étant relié au seul port artériel (Px2) alors que le premier port (Pm1) et le deuxième port (Pm2) sont respectivement isolés vis-à-vis du port artériel (Px2), les ports veineux et artériel (Px1, Px2) étant alors également isolés l'un de l'autre.

10. Dispositif d'interface selon l'une quelconque des revendications 1 à 8 combinée à la revendication 6, comportant également un port de restitution (Pr2) porté par ledit ensemble mobile (1a), le dispositif d'interface étant adapté pour sélectivement adopter une configuration de restitution veineuse (P5, Etp5) distincte des autres configurations du dispositif (P1, P6, P2, P3, Etp1 ,Etp4), dans cette configuration de restitution veineuse (P5, Etp5), le port de restitution (Pr2) est relié audit deuxième port (Pm2) pour pouvoir injecter un fluide de restitution vers l'équipement externe qui comprend une machine à hémodialyse (2), le premier port (Pm1) étant alors relié audit port veineux (Px1) et isolé de tous les autres ports du dispositif d'interface, et le port artériel (Px2) étant au moins isolé vis-à-vis du port veineux (Px1), du premier port (Pm1) et du deuxième port (Pm2).

11. Dispositif d'interface selon la revendication 10, dans lequel le dispositif d'interface (1) est adapté pour sélectivement adopter une configuration de restitution artérielle (P4, Etp6B) distincte des autres configurations du dispositif (P1, P6, P2, P3, P5, Etp1,Etp4, Etp5), dans cette configuration de restitution artérielle (P4, Etp6B), le port de restitution (Pr2) est relié audit deuxième port (Pm2) pour pouvoir injecter un fluide de restitution vers l'équipement externe qui comprend une machine à hémodialyse (2), le premier port (Pm1) étant alors relié audit port artériel (Px2) et isolé de tous les autres ports du dispositif d'interface, et le port veineux (Px1) étant au moins isolé vis-à-vis du port artériel (Px2), du premier port (Pm1) et du deuxième port (Pm2).

12. Dispositif d'interface selon l'une quelconque des revendications 10 ou 11, dans lequel le dispositif d'interface est adapté pour que lorsqu'il se trouve dans sa première configuration (P1, Etp1), son port de restitution (Pr2) est alors relié à l'un au moins desdits du premier port (Pm1) et du deuxième port (Pm2).

13. Dispositif d'interface selon l'une quelconque des revendications 1 à 12, dans lequel le dispositif d'interface (1) comporte un mécanisme de commande motorisé et agencé pour déplacer l'ensemble mobile (1a) par rapport au stator (1b) et ainsi commander le passage du dispositif de l'une de ses configurations vers une autre de ses configurations.

14. Ensemble d'interfaçage (100) comportant un dispositif d'interface (1) selon l'une quelconque des revendications 1 à 13 et une connectique d'interface (10) comportant une prise (10a) et une pluralité de tubes souples (10b) présentant chacun une extrémité reliée à la prise (10a) et une extrémité portant au moins un raccord de connexion (10c), chaque raccord de connexion (10c) donné étant destiné à connecter fluidiquement le tube souple portant ce raccord de connexion donné (10c) à l'un desdits ports de l'équipement externe (2) qui correspond à ce raccord de connexion donné (10c), la prise (10a) étant agencée pour être reliée mécaniquement et de manière amovible audit dispositif d'interface (1) de telle manière que lorsque la prise (10a) est reliée mécaniquement audit dispositif d'interface (1), chacun des tubes souples (10b) de la pluralité de tube souples est alors fluidiquement relié à un seul des ports portés par ledit ensemble mobile (1a) qui lui correspond.

15. Ensemble d'interfaçage (100) selon la revendication 14, dans lequel la prise (10a) et l'ensemble mobile (1a) du dispositif d'interface (1) sont conformées pour que lorsque la prise (10a) est reliée mécaniquement audit dispositif d'interface (1), cette prise (10a) est alors assujettie en déplacement avec l'ensemble mobile (1a) lors du déplacement de cet ensemble mobile (1a) par rapport au stator (1b).

16. Ensemble d'interfaçage (100) selon la revendication 15, dans lequel la connectique d'interface (10) comporte un indicateur (10e) de position de la prise, visible depuis l'extérieur de la connectique d'interface, pour informer un opérateur d'une position courante de la prise par rapport au stator lorsque la prise (10a) est reliée mécaniquement audit dispositif d'interface (1).

17. Ensemble d'interfaçage (100) selon l'une quelconque des revendications 14 à 16, dans lequel la connectique d'interface (10) comporte au moins un verrou mécanique (10f) agencé :
- pour interdire l'écartement relatif de la prise (10a) vis-à-vis du dispositif d'interface (1) lorsque la prise (10a) est reliée mécaniquement audit dispositif d'interface (1) et que cette prise (10a) est éloignée d'une position relative prédéterminée de la prise (10a) par rapport au stator (1b) ; et
- pour autoriser l'écartement relatif de la prise (10a) vis-à-vis du dispositif d'interface (1) lorsque la prise (10a) est reliée mécaniquement audit dispositif d'interface (1) et que cette prise (10a) se trouve dans ladite position relative prédéterminée de la prise (10a) par rapport au stator (1b).

18. Ensemble d'interfaçage (100) selon l'une quelconque des revendications 14 à 17, dans lequel ladite prise (10a) présente une pluralité de ports mâles chacun orienté pour pénétrer dans un des ports (Pm1, PR2, Pm2, PB1x) qui lui correspond et qui est porté par l'ensemble mobile (1a), réalisant ainsi une pluralité de connexions fluidiques entre les ports mâles portés par la prise (10a) et les ports correspondants portés par l'ensemble mobile (1a).

19. Ensemble d'interfaçage (100) selon l'une quelconque des revendications 14 à 18, dans lequel ladite prise (10a) et lesdits tubes souples sont en matériaux polymères alors que l'un au moins parmi l'ensemble mobile (1a) et le stator (1b) est au moins partiellement constitué en un matériau métallique.

20. Système d'hémodialyse (0) comprenant un ensemble d'interface (100) selon la revendication 14 comprenant le dispositif d'interface selon la revendication 6 et un équipement externe qui comprend une machine à hémodialyse (2), le premier port (Pm1) du dispositif d'interface (1) étant relié de manière amovible au port de sortie (M1) de la machine à hémodialyse (2) via la connectique d'interface (10) et le deuxième port (Pm2) du dispositif d'interface (1) étant relié de manière amovible au port d'entrée (M2) de la machine à hémodialyse via la connectique d'interface (10), la machine à hémodialyse comportant une pompe (M) agencée pour faire circuler des fluides de son port d'entrée (M2) vers son port de sortie (M1), le système d'hémodialyse comprenant également un tube veineux (X1) et un tube artériel (X2), le tube veineux (X1) étant reliée au port veineux (Px1) du dispositif d'interface (1), ce tube veineux (X1) étant destiné à être relié au système circulatoire (3) d'un patient pour transférer, via le dispositif d'interface (1) et via la connectique d'interface (10), du sang de la machine à hémodialyse (2) vers le système circulatoire (3), le tube artériel (X2) étant relié au port artériel (X2) du dispositif d'interface (1), ce tube artériel (X2) étant destiné à être relié audit système circulatoire (3) du patient pour transférer, via le dispositif d'interface (1) et via la connectique d'interface (10), du sang du système circulatoire (3) vers la machine à hémodialyse (2).

21. Système d'hémodialyse selon la revendication 20, dans lequel le système comporte une seringue d'aspiration (B1x) de fluide de verrouillage reliée au troisième port (Pblx) via la connectique d'interface (10) pour pouvoir aspirer du fluide de verrouillage provenant du tube artériel (X2).

22. Connectique d'interface (10) pour relier un équipement externe (2) à un dispositif d'interface (1) selon l'une quelconque des revendications 1 à 9 pour injecter un fluide vers un patient, **caractérisée en ce qu'**elle comporte une prise (10a) et une pluralité de tubes souples (10b) présentant chacun une extrémité reliée à la prise (10a) et une extrémité portant au moins un raccord de connexion (10c), chaque raccord de connexion (10c) donné étant destiné à connecter fluidiquement le tube souple portant ce raccord de connexion donné (10c) à un port de l'équipement externe (2) qui correspond à ce raccord de connexion donné (10c), la prise (10a) présentant une pluralité de ports mâles débouchant chacun vers une face de la prise et chacun desdits tubes souples étant relié fluidiquement à un seul desdits ports mâles qui lui correspond et inversement, chacun desdits ports mâles étant relié à un seul desdits tubes souples qui lui correspond.

## Patentansprüche

1. Schnittstellenvorrichtung (1) zwischen einem externen Gerät (2) und zumindest einem Venenschlauch (X1), der dazu bestimmt ist, mit einem Patientensystem (3) verbunden zu werden, um Fluid von dem Gerät zu dem Patientensystem zu leiten, wobei die Schnittstellenvorrichtung (1) zumindest Folgendes umfasst:
- einen ersten Port (Pm1), der dafür angepasst ist, mit einem Ausgangsport (M1) des externen Geräts (2) verbunden zu werden;
- einen Venenport (Px1), der dazu dient, Fluid zu dem Venenschlauch (X1) hin einzuspritzen; wobei die Schnittstellenvorrichtung dafür angepasst ist, selektiv eine erste Konfiguration (P1 ; Etp1) anzunehmen, in welcher der Fluiddurchgang zwischen dem ersten Port (Pm1) und dem Venenport (Px1) verhindert wird, und eine zweite Konfiguration (P6; Etp4) anzunehmen, in welcher der erste Port (Pm1) mit dem Venenport (Px1) verbunden ist, um den Fluiddurchgang von dem ersten Port (Pm1) zu dem Venenport (Px1) zu ermöglichen, **dadurch gekennzeichnet, dass**:
- die Schnittstellenvorrichtung (1) einen dritten Port (PB1x) enthält und dafür angepasst ist, selektiv eine Venensperre-Zugangskonfiguration (P3; Etp3b) anzunehmen, die sich von der ersten und der zweiten Konfiguration (P1, P6; Etp1; Etp4) unterscheidet, wobei in dieser Venensperre-Zugangskonfiguration (P3; Etp3b) der dritte Port (Pb1x) mit dem Venenport (Px1) verbunden ist, während der erste Port (Pm1) gegenüber dem Venenport (Px1) isoliert ist; und dass
- der Venenport (Px1) von einem Fixteil der Schnittstellenvorrichtung getragen wird, wobei der erste Port (Pm1) und der dritte Port (PB1x) von einer mobilen Anordnung (1a) der Schnittstellenvorrichtung (1) getragen werden, die in Bezug auf das Fixteil (1b) beweglich ist, wobei die Schnittstellenvorrichtung dafür ausgelegt ist, durch eine Relativverschiebung der mobilen Anordnung (1a) in Bezug auf das Fixteil von einer ihrer Konfigurationen in eine andere ihrer Konfigurationen zu wechseln.

2. Schnittstellenvorrichtung (1) nach Anspruch 1, wobei es sich bei der mobilen Anordnung um ein drehbar zu dem Fixteil angebrachtes Drehglied handelt.

3. Schnittstellenvorrichtung (1) nach einem der Ansprüche 1 oder 2, wobei die mobile Anordnung (1a) eine von außerhalb der Schnittstellenvorrichtung (1) sichtbare Seite (F1) aufweist und jeder der von der mobilen Anordnung (1a) getragenen Ports in besagte Seite ausmündet.

4. Schnittstellenvorrichtung (1) nach Anspruch 3, wobei die Seite (F1) der mobilen Anordnung (1a) und die von der mobilen Anordnung getragenen Ports zur Gänze innerhalb einer Ausnehmung des Fixteils angeordnet sind.

5. Schnittstellenvorrichtung nach einem der Ansprüche 1 bis 4, wobei es sich bei den von der mobilen Anordnung getragenen Ports um weibliche Ports handelt, die in eine für besagte weibliche Ports gemeinsame Richtung (Dx) hin ausmünden.

6. Schnittstellenvorrichtung (1) nach einem der Ansprüche 1 bis 5, ferner umfassend einen von der mobilen Anordnung der Schnittstellenvorrichtung (1) getragenen zweiten Port (Pm2), wobei die Schnittstellenvorrichtung weiterhin dafür angepasst ist, dass in besagter Venensperre-Zugangskonfiguration der erste Port (Pm1) und der zweite Port (Pm2) jeweils gegenüber dem Venenport isoliert sind.

7. Schnittstellenvorrichtung (1) nach Anspruch 6, die dafür angepasst ist, eine Schnittstelle zwischen dem externen Gerät, das eine Hämodialysemaschine (2) umfasst, und einem Arterienschlauch (X2) zu bilden, der dazu bestimmt ist, mit dem Patientensystem (3) verbunden zu werden, um Fluid von dem Patientensystem zu der Hämodialysemaschine zu leiten, wobei der zweite Port (Pm2) dafür angepasst ist, mit einem Eingangsport (M2) der Hämodialysemaschine verbunden zu werden, wobei die Schnittstellenvorrichtung (1) ferner einen von dem Fixteil getragenen Arterienschlauch (Px2) umfasst, um Patientenfluid (hier handelt es sich bei dem Fluid um Patientenblut) aus dem Arterienschlauch (X2) aufzunehmen; wobei die Schnittstellenvorrichtung (1) ferner dafür angepasst ist, den Fluiddurchgang zwischen dem zweiten Port (Pm2) und dem Arterienport (Px2) zu verhindern, wenn sich die Vorrichtung in der ersten Konfiguration (P1 ; Etp1) befindet und den Fluiddurchgang zwischen dem zweiten Port (Pm2) und dem Arterienport (Px2) zu erlauben, wenn sich die Vorrichtung in der zweiten Konfiguration (P6 ; Etp4) befindet.

8. Schnittstellenvorrichtung (1) nach einem der Ansprüche 1 bis 7, ferner dafür angepasst, über einen internen Kreislauf (A10) der Schnittstellenvorrichtung (1) den ersten Port (Pm1) mit zumindest einem anderen der von der mobilen Anordnung (1a) getragenen Ports (PR2, PB1x) in Verbindung zu setzen, wenn sich die Schnittstellenvorrichtung in ihrer ersten Konfiguration (P1) befindet, und die Verbindung zwischen dem ersten Port (Pm1) und den anderen von der mobilen Anordnung (1a) getragenen Ports (PR2, PB1x, Pm2) über diesen internen Kreislauf (A10) zu verhindern, wenn sich die Schnittstellenvorrichtung in ihrer zweiten Konfiguration (P6 ; Etp4) befindet.

9. Schnittstellenvorrichtung nach einem der Ansprüche 1 bis 8 in Kombination mit Anspruch 6, die dafür angepasst ist, selektiv eine Arteriensperre-Zugangskonfiguration (P2 ; Etp3a) anzunehmen, welche sich von der ersten und der zweiten Konfiguration (P1, P6; Etp1 ; Etp4) sowie von der Venensperre-Zugangskonfiguration (P3; Etp3b) unterscheidet, wobei in der Arteriensperre-Zugangskonfiguration (P2 ; Etp3a) der dritte Port (Pb1x) nur mit dem Arterienport (Px2) verbunden ist, während der erste Port (Pm1) und der zweite Port (Pm2) jeweils gegenüber dem Arterienport (Px2) isoliert sind, wobei der Venen- und der Arterienport (Px1, Px2) gegeneinander isoliert sind.

10. Schnittstellenvorrichtung nach einem der Ansprüche 1 bis 8 in Kombination mit Anspruch 6, ferner enthaltend einen von der mobilen Anordnung (1a) getragenen Restitutionsport (Pr2), wobei die Schnittstellenvorrichtung dafür angepasst ist, selektiv eine Venenrestitutionskonfiguration anzunehmen, die sich von den anderen Konfigurationen (P1, P6, P2, P3, Etp1, Etp4) der Vorrichtung unterscheidet, wobei in der Venenrestitutionskonfiguration (P5, Etp5) der Restitutionsport (Pr2) mit dem zweiten Port (Pm2) verbunden ist, um das Einspritzen eines Restituionsfluids zu dem externen Gerät hin zu ermöglichen, das eine Hämodialysemaschine (2) umfasst, wobei der erste Port (Pm1) nun mit dem Venenport (Px1) verbunden ist und dabei gegenüber allen anderen Ports der Schnittstellenvorrichtung isoliert ist, und der Arterienport (Px2) zumindest gegenüber dem Venenport (Px1), dem ersten Port (Pm1) und dem zweiten Port (Pm2) isoliert ist.

11. Schnittstellenvorrichtung nach Anspruch 10, wobei die Schnittstellenvorrichtung (1) dafür angepasst ist, selektiv eine Arterienrestitutionskonfiguration (P4, Etp6B) anzunehmen, die sich von den anderen Konfigurationen (P1, P6, P2, P3, P5, Etp1, Etp4, Etp5) der Vorrichtung unterscheidet, wobei in der Arterienrestitutionskonfiguration (P4, Etp6B) der Restitutionsport (Pr2) mit dem zweiten Port (Pm2) verbunden ist, um das Einspritzen eines Restituionsfluids zu dem externen Gerät hin zu ermöglichen, das eine Hämodialysemaschine (2) umfasst, wobei der erste Port (Pm1) nun mit dem Arterienport (Px2) verbunden ist und dabei gegenüber allen anderen Ports der Schnittstellenvorrichtung isoliert ist, und der Venenport (Px1) zumindest gegenüber dem Arterienport (Px2), dem ersten Port (Pm1) und dem zweiten Port (Pm2) isoliert ist.

12. Schnittstellenvorrichtung nach einem der Ansprüche 10 oder 11, wobei die Schnittstellenvorrichtung dafür angepasst ist, dass, wenn sie sich in ihrer ersten Konfiguration (P1, Etp1) befindet, ihr Restitutionsport (Pr2) mit zumindest dem ersten Port (Pm1) und/oder dem zweiten Port (Pm2) verbunden ist.

13. Schnittstellenvorrichtung nach einem der Ansprüche 1 bis 12, wobei die Schnittstellenvorrichtung (1) einen motorisierten Steuermechanismus enthält und dafür ausgelegt ist, die mobile Anordnung (1a) in Bezug auf das Fixteil (1b) zu bewegen und dadurch die Überführung der Vorrichtung von einer ihrer Konfigurationen in eine andere ihrer Konfigurationen anzusteuern.

14. Schnittstellenanordnung (100) enthaltend eine Schnittstellenvorrichtung (1) nach einem der Ansprüche 1 bis 13 und ein Schnittstellenanschlusssystem (10) mit einer Steckverbindung (10a) und einer Mehrzahl von flexiblen Schläuchen (10b), die jeweils ein mit der Steckverbindung (10a) verbundenes Ende sowie ein zumindest ein Anschlussstück (10c) tragendes Ende aufweisen, wobei jedes Anschlussstück (10c) dafür bestimmt ist, den flexiblen Schlauch, der besagtes Anschlussstück (10c) trägt, mit einem der Ports des externen Geräts (2), welcher dem Anschlussstück (10c) entspricht, in Fluidverbindung zu setzen, wobei die Steckverbindung (10a) dafür ausgelegt ist, derart mechanisch und lösbar mit der Schnittstellenvorrichtung (1) verbunden zu werden, dass, wenn die Steckverbindung (10a) mechanisch mit der Schnittstellenvorrichtung (1) verbunden ist, jeder der flexiblen Schläuche (10b) aus der Mehrzahl von flexiblen Schläuchen jeweils mit nur einem, ihm entsprechenden Port aus den Ports, die von der mobilen Anordnung (1a) getragen werden, in Fluidverbindung steht.

15. Schnittstellenanordnung (100) nach Anspruch 14, wobei die Steckverbindung (10a) und die mobile Anordnung (1a) der Schnittstellenvorrichtung (1) derart ausgebildet sind, dass, wenn die Steckverbindung (10a) mechanisch mit der Schnittstellenvorrichtung (1) verbunden ist, besagte Steckverbindung (10a) während der Verfahrbewegung der mobilen Anordnung (1a) in Bezug auf das Fixteil (1b) gemeinsam mit der mobilen Anordnung (1a) mitbewegt wird.

16. Schnittstellenanordnung (100) nach Anspruch 15, wobei das Schnittstellenanschlusssystem (10) einen von außerhalb des Schnittstellenanschlusssystems sichtbaren Positionsanzeiger (10e) der Steckverbindung enthält, der dazu dient, einen Bediener über eine aktuelle Position der Steckverbindung in Bezug auf das Fixteil zu informieren, wenn die Steckverbindung (10a) mechanisch mit der Schnittstellenvorrichtung (1) verbunden ist.

17. Schnittstellenanordnung (100) nach einem der Ansprüche 14 bis 16, wobei das Schnittstellenanschlusssystem (10) zumindest eine mechanische Sperre (10f) enthält, die dafür ausgelegt ist:
- das relative Sich-Entfernen der Steckverbindung (10a) gegenüber der Schnittstellenvorrichtung (1) zu verhindern, wenn die Steckverbindung (10a) mechanisch mit der Schnittstellenvorrichtung (1) verbunden ist und die Steckverbindung (10a) von einer vorbestimmten relativen Position der Steckverbindung (10a) in Bezug auf das Fixteil (1b) entfernt wird; und
- das relative Sich-Entfernen der Steckverbindung (10a) gegenüber der Schnittstellenvorrichtung (1) zu erlauben, wenn die Steckverbindung (10a) mechanisch mit der Schnittstellenvorrichtung (1) verbunden ist und sich die Steckverbindung (10a) in der vorbestimmten relativen Position der Steckverbindung (10a) in Bezug auf das Fixteil (1b) befindet.

18. Schnittstellenanordnung (100) nach einem der Ansprüche 14 bis 17, wobei die Steckverbindung (10a) eine Mehrzahl von männlichen Ports aufweist, die derart ausgerichtet sind, dass sie jeweils in einen der von der mobilen Anordnung (1a) getragenen entsprechenden Ports (Pm1, PR2, Pm2, PB1x) eingesteckt werden können, wodurch eine Mehrzahl von Fluidverbindungen zwischen den von der Steckverbindung (10a) getragenen männlichen Ports und den von der mobilen Anordnung (1a) getragenen entsprechenden Ports hergestellt wird.

19. Schnittstellenanordnung (100) nach einem der Ansprüche 14 bis 18, wobei die Steckverbindung (10a) und die flexiblen Schläuche aus Polymerwerkstoffen gebildet sind, während die mobile Anordnung (1a) und/oder das Fixteil (1b) zumindest teilweise aus einem metallischen Werkstoff gefertigt sind.

20. Hämodialysesystem (0) umfassend eine Schnittstellenanordnung (100) nach Anspruch 14, welche die Schnittstellenvorrichtung nach Anspruch 6 und ein externes Gerät mit einer Hämodialysemaschine (2) umfasst, wobei der erste Port (Pm1) der Schnittstellenvorrichtung (1) über das Schnittstellenanschlusssystem (10) lösbar mit dem Ausgangsport (M1) der Hämodialysemaschine (2) verbunden ist und der zweite Port (Pm2) der Schnittstellenvorrichtung (1) über das Schnittstellenanschlusssystem (10) lösbar mit dem Eingangsport (M1) der Hämodialysemaschine (2) verbunden ist, wobei die Hämodialysemaschine eine Pumpe (M) enthält, die für den Transport von Fluiden von dem Eingangsport (M2) zu dem Ausgangsport (M1) ausgelegt ist, wobei das Hämodialysesystem ferner einen Venenschlauch (X1) und einen Arterienschlauch (X2) umfasst, wobei der Venenschlauch (X1) mit dem Venenport (Px1) der Schnittstellenvorrichtung (1) verbunden ist, wobei der Venenschlauch (X1) dazu bestimmt ist, mit dem Kreislaufsystem (3) eines Patienten verbunden zu werden, um über die Schnittstellenvorrichtung (1) und über das Schnittstellenanschlusssystem (10) Blut von der Hämodialysemaschine (2) zu dem Kreislaufsystem (3) zu leiten, wobei der Arterienschlauch (X2) mit dem Arterienport (X2) der Schnittstellenvorrichtung (1) verbunden ist, wobei der Arterienschlauch (X2) dazu bestimmt ist, mit dem Patientenkreislaufsystem (3) verbunden zu werden, um über die Schnittstellenvorrichtung (1) und über das Schnittstellenanschlusssystem (10) Blut von dem Kreislaufsystem (3) zu der Hämodialysemaschine (2) zu leiten.

21. Hämodialysesystem nach Anspruch 20, wobei das System eine Aspirationsspritze (B1x) zum Absaugen von Sperrfluid enthält, welche über das Schnittstellenanschlusssystem (10) mit dem dritten Port (Pb1x) verbunden ist, um ein Absaugen von Sperrfluid aus dem Arterienschlauch (X2) zu ermöglichen.

22. Schnittstellenanschlusssystem (10) zur Verbindung eines externen Geräts (2) mit einer Schnittstellenvorrichtung (1) nach einem der Ansprüche 1 bis 9, um ein Fluid zu einem Patienten hin einzuspritzen, **dadurch gekennzeichnet, dass** das System eine Steckverbindung (10a) und eine Mehrzahl von flexiblen Schläuchen (10b) enthält, die jeweils ein mit der Steckverbindung (10a) verbundenes Ende sowie ein zumindest ein Anschlussstück (10c) tragendes Ende aufweisen, wobei jedes Anschlussstück (10c) dafür bestimmt ist, den flexiblen Schlauch, der das Anschlussstück (10c) trägt, mit einem Port des externen Geräts (2), der dem Anschlussstück (10c) entspricht, in Fluidverbindung zu setzen, wobei die Steckverbindung (10a) eine Mehrzahl von männlichen Ports aufweist, die zu einer Seite der Steckverbindung hin ausmünden und jeder der flexiblen Schläuche mit nur einem ihm entsprechenden männlichen Port aus der Mehrzahl von männlichen Ports in Fluidverbindung steht, und umgekehrt jeder der männlichen Ports mit nur einem ihm entsprechenden flexiblen Schlauch verbunden ist.

## Claims

1. An interface device (1) between external equipment (2) and at least a venous tube (X1) for connecting to a patient system (3) in order to transfer fluid from the equipment to the patient system, the interface device (1) comprising at least:
· a first port (Pm1) adapted to be connected to an outlet port (M1) of the external equipment (2); and
· a venous port (Px1) for injecting the fluid into the venous tube (X1);
· the interface device being adapted to adopt selectively a first configuration (P1; Etp1) in which fluid is prevented from passing between the first port (Pm1) and the venous port (Px1) and a second configuration (P6; Etp4) in which the first port (Pm1) is connected to the venous port (Px1) to allow fluid to pass from the first port (Pm1) to the venous port (Px1), the interface device (1) being **characterized in that**:
· it includes a third port (PB1x) and is adapted to adopt selectively a venous lock access configuration (P3; Etp3b) that is different from said first and second configurations (P1, P6; Etp1; Etp4), the third port (Pblx) being connected in this venous lock access configuration (P3; Etp3b) to the venous port (Px1) while the first port (Pm1) is isolated from the venous port (Px1); and **in that**
· the venous port (Px1) is carried by a stator of the interface device, the first port (Pm1) and the third port (PB1x) being carried by a movable assembly (1a) of the interface device (1) that is movable relative to the stator (1b), the interface device being arranged to pass from any one of its configurations to another one of its configurations by the movable assembly (1a) moving relative to the stator.

2. An interface device (1) according to claim 1, wherein the movable assembly is a rotor mounted to turn relative to said stator.

3. An interface device (1) according to claim 1 or claim 2, wherein the movable assembly (1a) has a face (F1) that is visible from the outside of the interface device (1), and each of the ports carried by the movable assembly (1a) opens out in said face.

4. An interface device (1) according to claim 3, wherein said face (F1) of the movable assembly (1a) and the ports carried by that movable assembly are located completely inside a recess of the stator.

5. An interface device according to any one of claims 1 to 4, wherein the ports carried by the movable assembly are female ports that open out in a direction (Dx) that is common to these female ports.

6. An interface device (1) according to any one of claims 1 to 5, also including a second port (Pm2) carried by said movable assembly of the interface device (1), the interface device further being adapted so that in said venous lock access configuration the first port (Pm1) and the second port (Pm2) are both isolated from the venous port.

7. An interface device (1) according to claim 6, adapted to form an interface between the external equipment, which comprises a hemodialysis machine (2), and an arterial tube (X2) for connecting to said patient system (3) in order to transfer fluid from the patient system to the hemodialysis machine, said second port (Pm2) being adapted to be connected to an inlet port (M2) of the hemodialysis machine, the interface device (1) also including an arterial port (Px2) carried by the stator to receive patient fluid coming from the arterial tube (X2);
· the interface device (1) further being adapted to prevent fluid from passing between the second port (Pm2) and the arterial port (Px2) when the device is in its first configuration (P1; Etp1) and to allow fluid to pass between the second port (Pm2) and the arterial port (Px2) when the device is in its second configuration (P6; Etp4).

8. An interface device (1) according to any one of claims 1 to 7, further adapted to put the first port (Pm1) into communication via an internal circuit (A10) of the interface device (1) with at least one other one of the ports (PR2, PB1x) carried by the movable assembly (1a) when the interface device is in its first configuration (P1), and to prevent communication via the internal circuit (A10) between the first port (Pm1) and the other ports (PR2, PB1x, Pm2) carried by the movable assembly (1a) when the interface device is in its second configuration (P6; Etp4).

9. An interface device according to any one of claims 1 to 8 in combination with claim 6, adapted to adopt selectively an arterial lock access configuration (P2; Etp3a) that is distinct from said first and second configurations (P1, P6; Etp1; Etp4) and from the venous lock access configuration (P3; Etp3b), in this arterial lock access configuration (P2; Etp3a) the third port (Pblx) being connected only to the arterial port (Px2) while the first port (Pm1) and the second port (Pm2) are both isolated from the arterial port (Px2), the venous and arterial ports (Px1, Px2) then also being isolated from each other.

10. An interface device according to any one of claims 1 to 8 in combination with claim 6, also including a restitution port (Pr2) carried by said movable assembly (1a), the interface device being adapted to adopt selectively a venous restitution configuration (P5, Etp5) distinct from the other configurations of the device (P1, P6, P2, P3, Etp1, Etp4), the restitution port (Pr2) being connected in this venous restitution configuration (P5, Etp5) to said second port (Pm2) in order to be able to inject a restitution fluid into the external equipment, which comprises a hemodialysis machine (2), the first port (Pm1) then being connected to said venous support (Px1) and being isolated from all of the other ports of the interface device, and the arterial port (Px2) being isolated at least from the venous port (Px1), from the first port (Pm1), and from the second port (Pm2).

11. An interface device according to claim 10, wherein the interface device (1) is adapted to adopt selectively an arterial restitution configuration (P4, Etp6B) that is distinct from the other configurations of the device (P1, P6, P2, P3, P5, Etp1, Etp4, Etp5), the restitution port (Pr2) being connected in this arterial restitution configuration (P4, Etp6B) to said second port (Pm2) in order to be able to inject a restitution fluid into the external equipment, which comprises a hemodialysis machine (2), the first port (Pm1) then being connected to said arterial port (Px2) and being isolated from all of the other ports of the interface device, and the venous port (Px1) being isolated at least from the arterial port (Px2), from the first port (Pm1), and from the second port (Pm2).

12. An interface device according to claim 10 or claim 11, wherein the interface device is adapted so that when it is in its first configuration (P1 Etp1), its restitution port (Pr2) is then connected to at least one of said first and second ports (Pm1, Pm2).

13. An interface device according to any one of claims 1 to 12, wherein the interface device (1) includes a motordriven control mechanism and is arranged for moving the movable assembly (1a) relative to the stator (1b), thereby causing the device to go from one of its configurations to another of its configurations.

14. An interface assembly (100) comprising an interface device (1) according to any one of claims 1 to 13 and interface connection means (10) comprising a plug (10a) and a plurality of flexible tubes (10b), each having one end connected to the plug (10a) and another end carrying at least one connection coupling (10c), each given connection coupling (10c) being for establishing a fluid flow connection between the flexible tube carrying the given connection coupling (10c) and a corresponding one of said ports of the external equipment (2), the plug (10a) being arranged to be mechanically connected in releasable manner to said interface device (1) in such a manner that when the plug (10a) is mechanically connected to said interface device (1), each of the flexible tubes (10b) of the plurality of flexible tubes is in fluid flow connection with only one of the ports carried by said movable assembly (1a) that corresponds thereto.

15. An interface assembly (100) according to claim 14, wherein the plug (10a) and the movable assembly (1a) of the interface device (1) are shaped so that when the plug (10a) is mechanically connected to said interface device (1), the plug (10a) is constrained to move together with the movable assembly (1a) when the movable assembly (1a) moves relative to the stator (1b).

16. An interface assembly (100) according to claim 15, wherein the interface connection means (10) include an indicator (10e) for indicating the position of the plug, which indicator is visible from outside the interface connection means so as to inform an operator about the current position of the plug relative to the stator when the plug (10a) is mechanically connected to said interface device (1).

17. An interface assembly (100) according to any one of claims 14 to 16, wherein the interface connection means (10) include at least one mechanical latch (10f) arranged:
· to prevent the plug (10a) and the interface device (1) from moving apart when the plug (10a) is mechanically connected to said interface device (1) and while the plug (10a) is not in a predetermined position relative to the stator (1b); and
· to allow the plug (10a) and the interface device (1) to move apart when the plug (10a) is mechanically connected to said interface device (1) and the plug (10a) is in said predetermined position relative to the stator (1b).

18. An interface assembly (100) according to any one of claims 14 to 17, wherein the plug (10a) presents a plurality of male ports, each oriented to penetrate into a corresponding respective one of the ports (Pm1, PR2, Pm2, PB1x) carried by the movable assembly (1a), thereby establishing a plurality of fluid flow connections between the male ports carried by the plug (10a) and the corresponding ports carried by the movable assembly (1a).

19. An interface assembly (100) according to any one of claims 14 to 18, wherein said plug (10a) and said flexible tubes are made out of polymer materials, while at least one of the movable assembly (1a) and the stator (1b) is made at least in part out of a metal material.

20. A hemodialysis system (0) comprising an interface assembly (100) according to claim 14 comprising both the interface device according to claim 6 and external equipment that comprises a hemodialysis machine (2), the first port (Pm1) of the interface device (1) being releasably connected to the outlet port (M1) of the hemodialysis machine (2) via the interface connection means (10) and the second port (Pm2) of the interface device (1) being releasably connected to the inlet port (M2) of the hemodialysis machine via the interface connection means (10), the hemodialysis machine including a pump (M) arranged to cause fluid to flow from its inlet port (M2) to its outlet port (M1), the hemodialysis machine also comprising a venous tube (X1) and an arterial tube (X2), the venous tube (X1) being connected to the venous port (Px1) of the interface device (1), the venous tube (X1) being for connection to a patient circulatory system (3) in order to transfer blood via the interface device (1) and via the interface connection means (10) from the hemodialysis machine (2) to the circulatory system (3), the arterial tube (X2) being connected to the arterial port (X2) of the interface device (1), the arterial tube (X2) being for connecting to said patient circulatory system (3) in order to transfer blood via the interface device (1) and via the interface connection means (10) from the circulatory system (3) to the hemodialysis machine (2).

21. A hemodialysis system according to claim 20, wherein the system includes a locking fluid suction syringe (B1x) connected to the third port (Pblx) via the interface connection means (10) so as to be able to suck locking fluid coming from the arterial tube (X2).

22. Interface connection means (10) for connecting external equipment (2) to an interface device (1) according to anyone of the claims 1 to 9 in order to inject fluid into a patient, the connection means being **characterized in that** they comprise a plug (10a) and a plurality of flexible tubes (10b), each having one end connected to the plug (10a) and another end carrying at least one connection coupling (10c), each given connection coupling (10c) being for putting the flexible tube carrying the given connection coupling (10c) into fluid flow connection with a corresponding port of the external equipment (2) that corresponds to the given connection coupling (10c), the plug (10a) having a plurality of male ports, each opening out in a face of the plug, and each of the flexible tubes being in fluid flow connection with a single corresponding one of said male ports, and conversely, each of said male ports being connected to a single corresponding one of said flexible tubes.
